# EUROPEAN PATENT APPLICATION

(11) **EP 4 331 472 A1**
(43) Date of publication of application: **06.03.2024**
(21) Application number: 22795365.0
(22) Date of filing: 18.03.2022
(51) Int. Cl.: A61B 5/00, A61B 5/243, A61B 5/318

(54) **BIOLOGICAL SIGNAL PROCESSING SYSTEM AND BIOLOGICAL SIGNAL MEASUREMENT SYSTEM**

(30) Priority: 26.04.2021 JP 2021074247
(71) Applicant: TDK Corporation, Tokyo 103-6128 (JP)
(72) Inventor: KURISU, Masafumi, Tokyo 103-6128 (JP)
(74) Representative: Epping - Hermann - Fischer
(86) International application number: PCT/JP2022/012715
(87) International publication number: WO 2022/230444

(57) **Abstract**

A biological signal processing system includes a segment identification unit configured to identify a segment including a time point of a processing target with respect to a biological signal whose period is divided into a plurality of segments in time, a processing control unit configured to select a processing method of processing the biological signal at the time point of the processing target on the basis of the segment identified by the segment identification unit, and a process execution unit configured to execute a process for the biological signal in the processing method selected by the processing control unit.

## Description

### [Technical Field]

The present disclosure relates to a biological signal processing system and a biological signal measurement system.

### [Background Art]

Biological signals are measured and the measured biological signals are processed.

Conventionally, there have been developments related to the processing of biological signals.

In technology described in Patent Document 1, a subject thought to have heart disease is estimated by quantitatively capturing features of measurement data obtained by a biomagnetic field measurement device, and further candidates for the disease are estimated on the basis of the measurement data of the subject. Thereby, the technology provides a biomagnetic field measurement device having a diagnosis assistance function of supporting a doctor's diagnosis, preventing the oversight of disease, and significantly shortening the diagnosis time (see paragraph 0004 of Patent Document 1). In the technology, for example, when the magnetic field emitted from the subject's living body is mainly a magnetic field emitted from the heart, feature parameters including an electric current direction at regular intervals near an R wave peak are calculated (see claims 1 and 3 of Patent Document 1).

In technology described in Patent Document 2, a method of segmenting each of a plurality of repetitive signal features in a biomedical signal, analyzing one or more segments to find values of a plurality of parameters describing the shape of the one or more of the segments, recording the values, and tracking changes in the values through the biomedical signal is proposed (see claim 1 of Patent Document 2). In the technology, for example, a template is defined on the basis of a shape of one or more waveforms (see claim 19 of Patent Document 2).

### [Citation List]

### [Patent Document]

[Patent Document 1]
   Japanese Unexamined Patent Application, First Publication No. 2003-144406
[Patent Document 2]
   Published Japanese Translation No. 2010-510851 of the PCT International Publication

### [Summary of Invention]

### [Technical Problem]

However, the conventional technology as described above may not be sufficient in terms of optimizing a process for each feature segment of the biological signal.

For example, in the waveform of a magnetocardiogram (MCG) signal, which is an example of a biological signal, an amplitude and a frequency differ according to each segment of a P wave, a QRS group, a T wave, or the like in one heartbeat. For this reason, when batch processing is performed on all segments of the magnetocardiogram signal waveform, a segment for which an appropriate process is implemented and a segment for which an appropriate process is not implemented are mixed and an optimum result may not be obtained.

Also, similar problems may also occur in other biological signals such as electrocardiogram (ECG) signals.

The present disclosure has been made in consideration of such circumstances and an objective of the present disclosure is to provide a biological signal processing system and a biological signal measurement system that can enable an appropriate process to be executed for each feature segment of a biological signal.

### [Solution to Problem]

According to an aspect, there is provided a biological signal processing system including: a segment identification unit configured to identify a segment including a time point of a processing target with respect to a biological signal whose period is divided into a plurality of segments in time; a processing control unit configured to select a processing method of processing the biological signal at the time point of the processing target on the basis of the segment identified by the segment identification unit; and a process execution unit configured to execute a process for the biological signal in the processing method selected by the processing control unit.

According to an aspect, there is provided a biological signal measurement system including: the biological signal processing system; and a biological signal measurement device configured to measure the biological signal.

### [Advantageous Effects of Invention]

According to the present disclosure, it is possible to execute an appropriate process for each feature segment of a biological signal in a biological signal processing system and a biological signal measurement system.

### [Brief Description of Drawings]

FIG. 1 is a diagram showing a schematic configuration of a biological signal measurement system including a biological signal processing system according to an embodiment.
FIG. 2 is a diagram showing an electrocardiogram signal corresponding to a magnetocardiogram signal which is an example of a biological signal according to the embodiment.
FIG. 3 is a diagram showing an example of a measurement unit of the biological signal measurement device according to the embodiment.
FIG. 4 is a diagram showing an example of the display of an electric current estimation calculation result according to the embodiment.
FIG. 5 is a diagram showing an example of the display of a magnetocardiogram signal and an electrocardiogram signal according to the embodiment.
FIG. 6 is a diagram showing an example of the display of results of executing a frequency filtering process of a frequency filtering method a1 for biological signals of all segments according to the embodiment.
FIG. 7 is a diagram showing an example of the display of results of executing a frequency filtering process of a frequency filtering method a2 for the biological signals of all the segments according to the embodiment.
FIG. 8 is a diagram showing an example of the display of a result of executing the frequency filtering process of the frequency filtering method a1 for a biological signal of a segment B1 according to the embodiment.
FIG. 9 is a diagram showing an example of the display of a result of executing the frequency filtering process of the frequency filtering method a2 for the biological signal of the segment B1 according to the embodiment.
FIG. 10 is a diagram showing an example of the display of a result of executing the frequency filtering process of the frequency filtering method a1 for a biological signal of a segment B2 according to the embodiment.
FIG. 11 is a diagram showing an example of the display of a result of executing the frequency filtering process of the frequency filtering method a2 for the biological signal of the segment B2 according to the embodiment.
FIG. 12 is a diagram showing an example of two different times with respect to the biological signal according to the embodiment.
FIG. 13 is a diagram showing an example of the display of a result of executing a calculation process of an electric current estimation calculation method b1 for the biological signal of the segment B1 according to the embodiment.
FIG. 14 is a diagram showing an example of the display of a result of executing a calculation process of an electric current estimation calculation method b2 for the biological signal of the segment B1 according to the embodiment.
FIG. 15 is a diagram showing an example of the display of a result of executing the calculation process of the electric current estimation calculation method b1 for the biological signal of the segment B2 according to the embodiment.
FIG. 16 is a diagram showing an example of the display of a result of executing the calculation process of the electric current estimation calculation method b2 for the biological signal of the segment B2 according to the embodiment.
FIG. 17 is a diagram showing an example of a processing procedure performed by the biological signal processing system in the biological signal measurement system according to the embodiment.
FIG. 18 is a diagram showing another example of the processing procedure performed by the biological signal processing system in the biological signal measurement system according to the embodiment.
FIG. 19 is a diagram showing an example of a measurement unit of a biological signal measurement device according to a modified example of the embodiment.

### [Description of Embodiments]

Hereinafter, embodiments of the present disclosure will be described with reference to the drawings.

### [Biological signal measurement system]

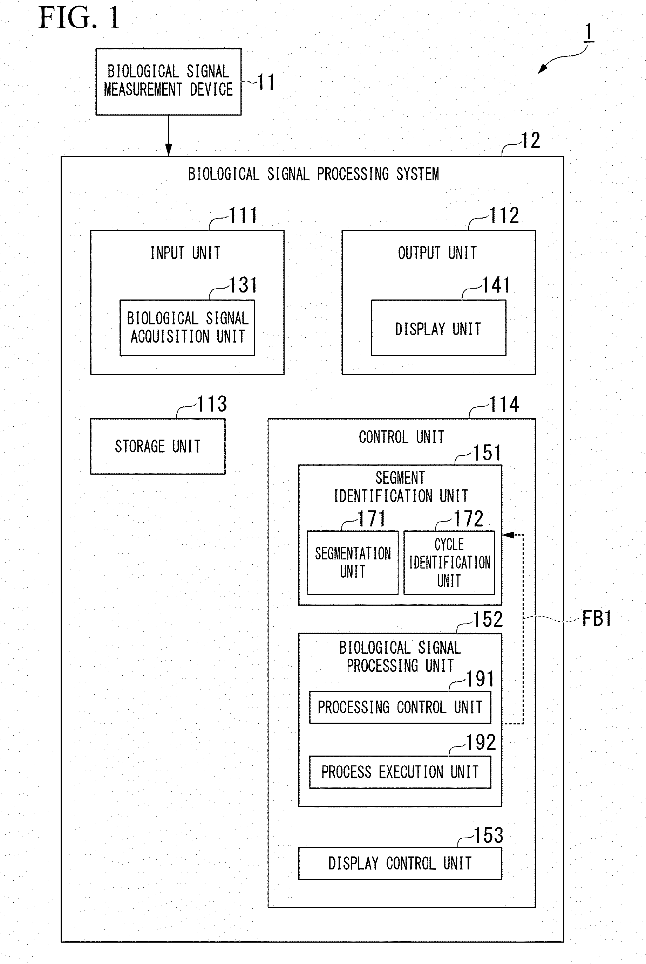

FIG. 1 is a diagram showing a schematic configuration of a biological signal measurement system 1 including a biological signal processing system 12 according to an embodiment.

The biological signal measurement system 1 includes a biological signal measurement device 11 and a biological signal processing system 12.

Although an example of a configuration in which the biological signal measurement device 11 and the biological signal processing system 12 are separate from each other is shown in the present embodiment, the biological signal processing system 12 may include the biological signal measurement device 11 in another configuration example.

### <Biological signal measurement device>

The biological signal measurement device 11 measures a biological signal.

In the present embodiment, the biological signal measurement device 11 includes a magnetocardiograph. The magnetocardiograph measures a biomagnetic field on the front surface (the surface including the belly) of an area from the shoulders to the torso of a human being (referred to as the upper body in the present embodiment for the convenience of description) and detects a magnetocardiogram signal, which is a measurement signal, as the biological signal. The biological signal measurement device 11 may measure biomagnetic fields of side surfaces (side surfaces of both sides) or the backside (the surface including the back) instead of the front surface with the magnetocardiograph.

Also, the present disclosure is not limited to the example of the present embodiment and the biological signal measurement device 11 may measure any part with the magnetocardiograph.

Also, the biological signal measurement device 11 may measure a plurality of parts of the same human being at the same time. In the description of the present embodiment, each of such a plurality of measurement paths is referred to as a channel.

For example, the biological signal measurement device 11 includes sensors that perform measurements for channels, and the sensors of these channels can obtain measurement results of the plurality of channels in one measurement process.

Here, the biological signal measurement device 11 may include any measurement instrument other than the magnetocardiograph and may measure any biological signal with the measurement instrument instead of a magnetocardiogram signal according to the present embodiment.

Examples of the biological signal measurement device 11 include an electrocardiograph and the electrocardiograph may measure a human electrocardiogram signal as a biological signal.

Also, for example, the biological signal measurement device 11 may simultaneously measure magnetocardiogram signals and electrocardiogram signals for the same human being.

The biological signal (the magnetocardiogram signal in the present embodiment) measured by the biological signal measurement device 11 is input to the biological signal processing system 12.

Here, the biological signal may be input to the biological signal processing system 12 in any method. As a specific example, the biological signal may be transmitted from the biological signal measurement device 11 to the biological signal processing system 12 by wired or wireless communication or the biological signal may be output from the biological signal measurement device 11, stored in a portable storage device, carried in the storage device, and input from the storage device to the biological signal processing system 12. The storage device may be, for example, a Universal Serial Bus (USB) memory or the like.

Also, the biological signal output from the biological signal measurement device 11 and input to the biological signal processing system 12 may be, for example, a measured raw signal, or may be a signal obtained by performing a certain process on a measured raw signal.

Also, the biological signal may be an analog signal or a digital signal. Although an example of a configuration in which the biological signal processed by the biological signal processing system 12 is converted into a digital signal (digital data) by the biological signal measurement device 11 or the biological signal processing system 12 and processed by the biological signal processing system 12 is shown in the present embodiment, a configuration in which the biological signal is processed as an analog signal by the biological signal processing system 12 may be used as another configuration example.

Also, for measurement by the magnetocardiograph, technology for obtaining a three-dimensional magnetic field distribution by arranging the sensors, obtaining a magnetic field distribution of vector quantities in three axial directions, or reconstructing an electric current source from magnetic field data (three-dimensional distribution estimation) or the like is being studied. For this reason, in a measurement process of the magnetocardiograph, a variety of three-dimensionally distributed information compared to electrocardiograms is expected and there is a need for technology for visualizing and displaying spatially distributed information in a better way.

In the present embodiment, it is also possible to respond to such a request.

### <Biological signal processing system>

The biological signal processing system 12 includes an input unit 111, an output unit 112, a storage unit 113, and a control unit 114.

The input unit 111 includes a biological signal acquisition unit 131.

The output unit 112 includes a display unit 141.

The control unit 114 includes a segment identification unit 151, a biological signal processing unit 152, and a display control unit 153.

The segment identification unit 151 includes a segmentation unit 171 and a cycle identification unit 172.

The biological signal processing unit 152 includes a processing control unit 191 and a process execution unit 192.

The input unit 111 receives an external input.

In the present embodiment, the input unit 111 inputs a biological signal output from the biological signal measurement device 11. As a specific example, the input unit 111 may input the biological signal by receiving the biological signal transmitted from the biological signal measurement device 11 or input the biological signal stored in the portable storage device from the storage device.

Also, the input unit 111 may have an operation unit operated by a user as an example and may input information corresponding to content of an operation performed by the user to the operation unit.

The biological signal acquisition unit 131 acquires a biological signal input by the input unit 111.

The biological signal acquisition unit 131 may store the acquired biological signal in the storage unit 113.

Here, when the biological signal input by the input unit 111 is an analog signal, for example, the biological signal acquisition unit 131 includes an analog to digital (A/D) conversion function and may convert the biological signal from an analog signal into a digital signal.

Also, when the biological signal processing system 12 is applied to real-time processing, the biological signal acquisition unit 131 acquires a biological signal in real-time. Also, even if the biological signal processing system 12 is not applied to real-time processing, the biological signal acquisition unit 131 may acquire a biological signal in real-time.

The output unit 112 externally provides an output.

The display unit 141 displays and outputs information about a result of processing the biological signal.

The display unit 141 has a screen such as a liquid crystal display (LCD), and displays and outputs information about the biological signal processing result on the screen. As another configuration example, the display unit 141 may print information about a result of processing the biological signal on paper.

Also, the output unit 112 may have a function of providing an output in other modes like an audio output and the like.

The storage unit 113 has a storage device such as a memory as an example and stores information.

The storage unit 113 stores, for example, information about an input biological signal, a result of processing the biological signal, and the like.

Also, the storage unit 113 stores information of a control program and the like as an example.

The control unit 114 performs various types of processes or control in the biological signal processing system 12.

In the present embodiment, the control unit 114 has a processor such as a central processing unit (CPU) and the processor executes a control program stored in the storage unit 113 to perform various types of processes or control.

Also, the processor includes a calculation device that performs various types of calculations.

### <<Segment identification>>

The segment identification unit 151 identifies a segment in the biological signal. In the present embodiment, the segment is a segment in time.

The segmentation unit 171 has a function of dividing the period of the biological signal into a plurality of segments. Any method may be used as a method in which the segmentation unit 171 divides a period of the biological signal into a plurality of segments.

The cycle identification unit 172 has a function of identifying a cycle of the biological signal. Any method may be used as a method in which the cycle identification unit 172 identifies the cycle of the biological signal.

Here, the method in which the segment identification unit 151 identifies a segment in the biological signal may be arbitrary.

Also, the segment identification unit 151 may use one or both of the function of the segmentation unit 171 and the function of the cycle identification unit 172 when identifying the segment in the biological signal.

Also, the segment identification unit 151 may identify the segment in the biological signal without using one or both of the function of the segmentation unit 171 and the function of the cycle identification unit 172. In this case, the functional units that are not used (here, one or both of the segmentation unit 171 and the cycle identification unit 172) may not be provided in the segment identification unit 151.

As an example, the segment identification unit 151 may identify a prescribed segment among a plurality of segments preset for the biological signal. In this case, the segmentation unit 171 and the cycle identification unit 172 may not be provided in the segment identification unit 151.

As another example, the segment identification unit 151 may divide the period of the biological signal into a plurality of segments with the segmentation unit 171 and identify a prescribed segment among the plurality of segments into which the period is divided. In this case, the cycle identification unit 172 may not be provided in the segment identification unit 151.

As another example, the segment identification unit 151 may identify the cycle of the biological signal with the cycle identification unit 172 and identify a prescribed segment in the biological signal on the basis of the identified cycle. In this case, the segmentation unit 171 may not be provided in the segment identification unit 151.

As another example, the segment identification unit 151 may identify the cycle of the biological signal with the cycle identification unit 172, divide the period of the biological signal into a plurality of segments with the segmentation unit 171, and identify a prescribed segment among the plurality of segments into which the period is divided.

As a specific example, the segmentation unit 171 may detect a feature quantity of the read biological signal and perform segmentation on the basis of the detected feature quantity. The feature quantity may be any feature quantity or a feature quantity of a waveform of the biological signal.

For example, the segmentation unit 171 may detect a peak of the read biological signal and perform segmentation on the basis of the detected peak. In this case, as an example, a pattern in which a peak appears in the biological signal of a measurement target is stored in the storage unit 113 in advance and the segmentation unit 171 may perform segmentation on the basis of the pattern and the peak detected from a measurement result (the biological signal).

As a specific example, the segmentation unit 171 may perform segmentation as designated according to the operation of the user.

As a specific example, the segmentation unit 171 may dynamically (in real-time) process the biological signal and perform segmentation. In this case, as an example, a model related to a dynamic feature quantity such as a change in the amplitude of a biological signal is stored in the storage unit 113 in advance and the segmentation unit 171 may perform segmentation on the basis of the model and the dynamic feature quantity detected from the measurement result (biological signal).

As a specific example, the segment identification unit 151 may identify a cycle with the cycle identification unit 172 for the biological signal that has been acquired dynamically (in real-time) and estimate a time point of the processing target on the basis of the identified cycle.

The segment identification unit 151 may perform segmentation or segment identification in a future time period (a future time period compared to a previous time period) in the biological signal on the basis of, for example, a segment in a previous time period in a biological signal. In the present embodiment, the biological signal is a signal in which a periodic waveform having a similar feature is repeated and a cycle (or a segment) in a certain time period can be predicted from a cycle (or a segment) in a previous time period earlier than the certain time period. For example, the segment identification unit 151 may identify the next cycle (or a segment in the next cycle) of the biological signal on the basis of a cycle (or a segment) of the biological signal of a previous cycle.

In this case, when it is determined that the cycle of the biological signal is gradually shortening, the segment identification unit 151 may estimate a cycle shorter than the previous cycle as the next cycle. In such a case, for example, the cycle of the human heartbeat of a measurement target may be gradually shortened.

On the other hand, when it is determined that the cycle of the biological signal is gradually increasing, the segment identification unit 151 may estimate a cycle longer than the previous cycle as the next cycle. In such a case, for example, the cycle of the human heartbeat of a measurement target may be gradually lengthened.

Also, for example, when a pattern having a relatively short cycle or a relatively long cycle is preset or determined once out of a prescribed number of times, the segment identification unit 151 may estimate a cycle (or a segment) on the basis of the pattern.

Also, the cycle identification unit 172 may identify the cycle using, for example, the number of samples, or may identify the cycle on the basis of an interval between peaks detected on the basis of data of the biological signal (biological data). In the present embodiment, the cycle identification unit 172 may identify the cycle on the basis of a result of analyzing the biological signal instead of the biological signal as an example.

The segment identification unit 151 may estimate a time at which information of a processing target (information about the biological signal) is located in the cycle on the basis of a cycle identified by the cycle identification unit 172. It is only necessary to use the time to identify a position in the cycle and the time may not necessarily be an absolute time. That is, the segment identification unit 151 may estimate information of a time point in one cycle (i.e., information based on a biological signal of a position) corresponding to the information of the processing target (the information about the biological signal).

Although a configuration in which the segment identification unit 151 identifies a segment on the basis of the biological signal of the processing target (a magnetocardiogram signal in the present embodiment) and the biological signal processing unit 152 processes the biological signal (the magnetocardiogram signal in the present embodiment) is shown in the present embodiment, a configuration in which the segment identification unit 151 identifies a segment on the basis of another biological signal (for example, an electrocardiogram signal measured simultaneously with a biological signal serving as a main processing target) related to a biological signal (the magnetocardiogram signal in the present embodiment) serving as the main processing target and the biological signal processing unit 152 processes the biological signal (the magnetocardiogram signal in the present embodiment) serving as the main processing target may be used as another configuration example.

### «Biological signal processing»

The biological signal processing unit 152 processes the biological signal.

The process execution unit 192 executes a process for the biological signal. In the present embodiment, the process execution unit 192 has a function of executing the process for the biological signal by switching a processing method between a plurality of different processing methods for the same type of process.

The processing control unit 191 controls the execution of the process with the process execution unit 192. In the present embodiment, the processing control unit 191 selects a processing method on the basis of the segment identified by the segment identification unit 151 and controls the process execution unit 192 so that the process execution unit 192 executes a process for the biological signal in the selected processing method. For example, the processing control unit 191 selects a different processing method for at least one segment as compared with other segments.

Here, in the present embodiment, three types of processes such as a frequency filtering process, an electric current estimation calculation process, and a region extraction process are exemplified as the same type of process.

Also, as a plurality of different processing methods related to each type of process, a plurality of different frequency filtering methods, a plurality of different electric current estimation calculation methods, and a plurality of different region extraction methods are exemplified. Also, names of the frequency filtering method, the electric current estimation calculation method, and the region extraction method are for the convenience of description and the present disclosure is not limited to these names.

### «Biological signal processing: frequency filtering method»

The frequency filtering method is a method of applying a prescribed frequency filtering process to a biological signal. A plurality of different frequency filtering methods are methods of applying frequency filtering processes having different features to biological signals.

The number of different frequency filtering methods may be any value greater than or equal to 2.

Corresponding relationships between the plurality of segments and the plurality of frequency filtering methods may be preset fixedly or may be variable. When the corresponding relationship is variable, for example, the initial content of the corresponding relationship may be preset or the corresponding relationship may not be set initially.

When the corresponding relationship is variable, the corresponding relationship may be automatically determined and set (together with the update setting) by the control unit 114 (for example, the processing control unit 191) according to a predetermined rule as an example, may be arbitrarily set (together with the update setting) according to the operation of the user, or may be set by both. The rule may be written in the control program or its parameters.

There may be cases where the same frequency filtering method is associated with two or more different segments.

For example, if a type of biological signal (a magnetocardiogram signal in the present embodiment) and a segment are determined in relation to the frequency filtering method, when the frequency filtering process suitable for the segment is identified (or estimated), the application of this frequency filtering process may be preset fixedly, but it is possible to arbitrarily change the frequency filtering process to be applied in accordance with the preference of the user or the like. Also, the control unit 114 (for example, the processing control unit 191) may automatically determine the frequency filtering process to be applied according to a rule based on a feature (for example, one or more of a frequency, an amplitude, and the like) of a signal component of a corresponding segment in an actual measurement signal (a biological signal).

As a frequency filtering method for each segment, for example, a frequency filtering process having a filtering feature for transmitting a signal component of a frequency band of interest in each segment and removing (for example, reducing) a signal component of another frequency band (particularly, a signal component of a frequency band with a large amplitude) is applied.

### «Biological signal processing: electric current estimation calculation method»

The electric current estimation calculation method is a method in which a prescribed calculation process is applied to a biological signal to estimate an electric current based on a biological signal. A plurality of different electric current estimation calculation methods are methods of applying different calculation processes to biological signals. In the present embodiment, the biological signal is a magnetocardiogram signal and a spatial filtering method of solving the inverse problem of estimating the signal source from the magnetocardiogram is used as the calculation method.

The number of different electric current estimation calculation methods may be any value greater than or equal to 2.

Also, as the calculation method, other calculation methods may be used instead of the electric current estimation calculation method.

Corresponding relationships between the plurality of segments and the plurality of electric current estimation calculation methods may be preset fixedly or may be variable. When the corresponding relationship is variable, for example, the initial content of the corresponding relationship may be preset or the corresponding relationship may not be set initially.

When the corresponding relationship is variable, the corresponding relationship may be automatically determined and set (together with the update setting) by the control unit 114 (for example, the processing control unit 191) according to a predetermined rule as an example, may be arbitrarily set (together with the update setting) according to the operation of the user, or may be set by both. The rule may be written in the control program or its parameters.

There may be cases where the same electric current estimation calculation method is associated with two or more different segments.

For example, if a type of biological signal (a magnetocardiogram signal in the present embodiment) and a segment are determined in relation to the electric current estimation calculation method, when the calculation process suitable for the segment is identified (or estimated), the application of this calculation process may be preset fixedly, but it is possible to arbitrarily change the calculation process to be applied in accordance with the preference of the user or the like. Also, the control unit 114 (for example, the processing control unit 191) may automatically determine the calculation process to be applied according to a rule based on a feature (for example, one or more of a frequency, an amplitude, and the like) of a signal component of a corresponding segment in an actual measurement signal (a biological signal).

### «Biological signal processing: region extraction method»

The region extraction method is a method of applying a region extraction process of extracting a biological signal of a prescribed region that is a processing target in a living body (a human being in the present embodiment) to a biological signal. A plurality of different region extraction methods are methods of applying different region extraction processes to biological signals. In the present embodiment, the plurality of different region extraction methods are methods in which the channels serving as the processing targets are different in measurement results (biological signals) of the plurality of channels. The number of channels serving as the processing targets in each region extraction method may be any value greater than or equal to 1.

The number of different region extraction methods may be any value of two or more.

Corresponding relationships between the plurality of segments and the plurality of region extraction methods may be preset fixedly or may be variable. When the corresponding relationship is variable, for example, the initial content of the corresponding relationship may be preset or the corresponding relationship may not be set initially.

When the corresponding relationship is variable, the corresponding relationship may be automatically determined and set (together with the update setting) by the control unit 114 (for example, the processing control unit 191) according to a predetermined rule as an example, may be arbitrarily set (together with the update setting) according to the operation of the user, or may be set by both. The rule may be written in the control program or its parameters.

There may be cases where the same region extraction method is associated with two or more different segments.

For example, if a type of biological signal (a magnetocardiogram signal in the present embodiment) and a segment are determined in relation to the region extraction method, when the region extraction suitable for the segment is identified (or estimated), the application of this region extraction process may be preset fixedly, but it is possible to arbitrarily change the region extraction process to be applied in accordance with the preference of the user or the like. Also, the control unit 114 (for example, the processing control unit 191) may automatically determine the region extraction process to be applied according to a rule based on a feature (for example, one or more of a frequency, an amplitude, and the like) of a signal component of a corresponding segment in an actual measurement signal (a biological signal).

### «Biological signal processing: selection of all processing methods»

In the present embodiment, the processing control unit 191 selects one region extraction method, one frequency filtering method, and one electric current estimation calculation method on the basis of the segment identified by the segment identification unit 151.

As an example, the process execution unit 192 executes the frequency filtering process applied in the frequency filtering method selected by the processing control unit 191 for the measurement result (biological signal) of the channel that is the processing target in the region extraction method selected by the processing control unit 191 and subsequently executes the calculation process applied in the electric current estimation calculation method selected by the processing control unit 191 for the biological signal for which the frequency filtering process has been executed.

As another example, the process execution unit 192 may first execute a frequency filtering process applied in the frequency filtering method selected by the processing control unit 191 for all channel measurement results (biological signals). Subsequently, the process execution unit 192 executes the calculation process applied in the electric current estimation calculation method selected by the processing control unit 191 for the channel that is the processing target in the region extraction method selected by the processing control unit 191 among the biological signals of all channels on which the frequency filtering process has been executed.

Although a case where each of three types of processing methods such as a plurality of different frequency filtering methods, a plurality of different electric current estimation calculation methods, and a plurality of different region extraction methods can be selected as the processing method is shown in the present embodiment, a configuration in which any one type of processing method among the frequency filtering method, the electric current estimation calculation method, and the region extraction method can be selected may be used as another configuration example or a configuration in which any two processing methods can be selected may be used as another configuration example.

Also, a configuration in which four or more types of processing methods can be selected as the processing method may be used.

Also, a selectable processing method is not limited to the processing method (the frequency filtering method, the electric current estimation calculation method, or the region extraction method) in the present embodiment and any processing method may be used as the selectable processing method.

Although a case where at least one type of processing method is switched on the basis of the segment identified by the segment identification unit 151 is shown in the present embodiment, the biological signal processing unit 152 (the processing control unit 191 or the process execution unit 192) further may have a function of processing the biological signal in the same processing method for all segments regardless of the segment of the biological signal.

### «Biological signal processing: feedback for segment identification»

The segment identification unit 151 may refer to a result of signal processing performed by the biological signal processing unit 152 when the segment in the biological signal is identified. As the result of the signal processing, for example, the result of executing a process of the frequency filtering method may be used, the result of executing the calculation process of the electric current estimation calculation method after the process of the frequency filtering method may be used, or both results may be used. In the result of the signal processing performed by the biological signal processing unit 152, a feature of the biological signal (for example, a feature such as a peak in each segment) may appear stronger than before the signal processing was performed, and it may be useful for identifying the segment in the biological signal.

Thus, the segment identification unit 151 may identify the segment on the basis of the result of executing a prescribed process for the biological signal. The prescribed process may be all or a part of the process executed by the biological signal processing unit 152.

For example, the segment identification unit 151 may identify the segment on the basis of a result of detecting noise of the biological signal.

As a specific example, the segmentation unit 171 may perform segmentation on the basis of the result of detecting the noise of the biological signal.

As a specific example, the cycle identification unit 172 may identify the cycle on the basis of the result of detecting the noise of the biological signal.

As a specific example, the segment identification unit 151 may estimate a time point of the processing target on the basis of the result of detecting the noise of the biological signal.

In these cases, the control unit 114 includes a function of a noise detection unit that detects noise contained in the biological signal. The function may be provided in the biological signal processing unit 152 as an example. The noise detection result may be, for example, a level of the noise or a waveform. The noise may be, for example, white noise.

The segment identification unit 151 may adjust a segment (for example, a predetermined segment or a segment of the division) on the basis of the noise detection result.

As a specific example, when a condition that a value related to noise in a certain segment is greater than or equal to a prescribed value (or a condition that the value related to the noise in the certain segment exceeds the prescribed value) is satisfied, the segment identification unit 151 may make an adjustment for narrowing one or both of both ends of the segment (a boundary of a point where a time value is smallest and a boundary of a point where a time value is largest).

Also, as a specific example, when a condition that the value related to the noise in the certain segment is less than the prescribed value (or a condition that the value related to the noise in the certain segment is less than or equal to the prescribed value) is satisfied, the segment identification unit 151 may make an adjustment for extending one or both of both ends of the segment (the boundary of the point where the time value is smallest and the boundary of the point where the time value is largest).

Here, as the value related to the noise, for example, the noise level may be used or a ratio of a noise level to a level of the biological signal on which the noise is carried (the biological signal in the present example) may be used.

In FIG. 1, an arrow FB1 schematically representing feedback from the biological signal processing unit 152 to the segment identification unit 151 is shown.

Also, feedback from the biological signal processing unit 152 to the segment identification unit 151 may not necessarily be performed.

### «Biological signal processing: segment for which process is unnecessary»

Also, the biological signal processing unit 152 may not perform the frequency filtering process and the calculation process of the electric current estimation calculation method for the biological signal, or may remove the biological signal of the segment in the frequency filtering process having a filtering feature for removing the biological signal of the segment, with respect to the segment for which the process is unnecessary, or may maintain a signal at a certain level (for example, a zero level or the like) by simply deleting the biological signal of the segment.

Also, the segment for which the process is unnecessary may be preset or may be set by the operation of the user as an example.

As another example, the biological signal processing unit 152 may determine that a segment other than one or more segments of interest is a segment for which the process is unnecessary. Also, the segment of interest may be preset or may be set by the operation of the user as an example.

### «Control of display»

The display control unit 153 controls a display mode when information about the biological signal is displayed by the display unit 141. In the present embodiment, the display control unit 153 controls the display mode of information about the biological signal of each segment in accordance with the segment identified by the segment identification unit 15. In this case, the display control unit 153 may control the display mode of a result of executing a process in the processing method in accordance with, for example, the processing method selected in the biological signal processing unit 152 (i.e., the processing method associated with each segment).

As the information related to the biological signal, for example, information of the biological signal (itself) or information of a result of executing a prescribed process for the biological signal may be used. The prescribed process may be a frequency filtering process or may be both a frequency filtering process and a calculation process of an electric current estimation calculation method.

For example, if information about a biological signal is displayed, the display control unit 153 may perform control so that information indicating the range of the segment is displayed for at least one of a plurality of segments when the information is about the plurality of segments. The range of the segment may be displayed, for example, by information of a line, a symbol, or the like indicating the entire range, may be displayed using a different color for each segment, or may be displayed by information of a boundary line indicating a boundary with other adjacent segments.

At this time, when the processing method is different in the two adjacent segments, the display control unit 153 may adjust (correct) one or both of information of one of these two segments (for example, a waveform or the like) and information of the other segment (for example, a waveform or the like) and perform control so that the information is smoothly concatenated at a boundary between the two segments.

When time-series information is displayed, the display control unit 153 may switch a time range that is a display target within the range for each segment. For example, when information about a biological signal is displayed, the display control unit 153 may perform control so that the information is displayed on a different screen display for each of the plurality of segments. As an example, the display control unit 153 may perform control so that only information of a specific segment is displayed on the screen and switch the screen display by switching a segment that is a display target.

For example, the display control unit 153 may perform control so that information about each segment is displayed for each segment. The information about each segment is not particularly limited and may include, for example, one or more items of information for identifying a segment and information for identifying a processing method applied to each segment.

Here, the information for identifying the segment may be a name, a number, or a mark of each segment.

Also, in the present embodiment, the information for identifying the processing method may be any one item or any two or three (all) items among information for identifying a frequency filtering method, information for identifying an electric current estimation calculation method, and information for identifying a region extraction method. The information for identifying these processing methods may be a name, a number, or a mark of each processing method or may be information indicating a feature of each processing method.

As an example, as a feature of the frequency filtering method, a frequency representing a filtering feature may be used. For example, a cutoff frequency may be used when a high-pass filter (HPF) or a low-pass filter (LPF) is applied.

When information is displayed in association with a region of a living body (the human body in the present embodiment), the display control unit 153 may switch a region to be displayed for each segment.

For example, when information is displayed in association with a region of a living body (the human body in the present embodiment) through two-dimensional display (planar display) or three-dimensional display (three-dimensional display), the display control unit 153 may perform control so that the information is displayed in a display mode focusing on information of a region extracted in the region extraction method corresponding to each segment for each segment. As the display mode focusing on the information of the region, for example, a display mode in which the information is displayed by cutting out the region, a display mode in which the information is displayed by expanding the region, or the like may be used.

Here, for a plurality of segments, the association of each segment, the name of the segment, a processing method applied to each segment (a frequency filtering method, an electric current estimation calculation method, or a region extraction method in the present embodiment), and the like may be stored as segment information in the storage unit 113. In this case, the display control unit 153 may control the display mode on the basis of the segment information.

### «Example of configuration related to control unit»

Also, the segment identification unit 151 (the segmentation unit 171 and the cycle identification unit 172), the biological signal processing unit 152 (the processing control unit 191 and the process execution unit 192), and the display control unit 153 are functional units exemplified to describe the functions of the control unit 114, and are not limited to the present embodiment, and the control unit 114 may have any function.

Although an example of a configuration in which the biological signal processing system 12 includes functional units (the input unit 111, the output unit 112, the storage unit 113, and the control unit 114) is shown in the example of FIG. 1, the functional units may be configured as a single device or may be configured to be distributed into two or more separate devices.

Also, the configuration of the functional units (the input unit 111, the output unit 112, the storage unit 113, and the control unit 114) of the biological signal processing system 12 shown in FIG. 1 is an example and is not limited to the present embodiment. The biological signal processing system 12 may have any functional unit.

Although a time point (a time axis value) of the biological signal is described as the time in the present embodiment, a sampling number or the like may be used instead of the time. For example, in sampling at regular time intervals, the progress of the sampling number and the progress of the time are proportional.

Also, as the time, the sampling number, or the like, for example, an absolute value may be used, or a relative value may be used. For example, in the present embodiment, if it is possible to identify a segment of a biological signal, any value may be used as the value of the time axis.

### [Example of biological signal]

Here, a magnetocardiogram signal, which is a biological signal that is a processing target in the present embodiment, will be described.

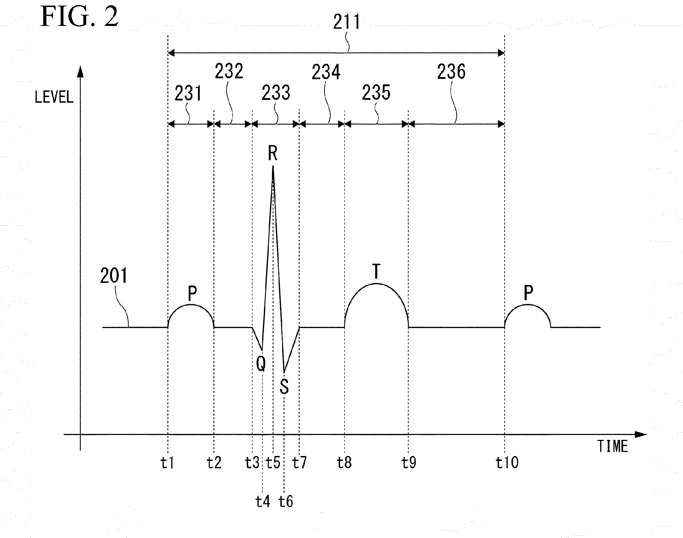

However, because the waveform of the magnetocardiogram signal is similar to the waveform of the electrocardiogram signal, which is the standard of the cardiac waveform, and can be regarded as similar to the waveform of the electrocardiogram signal, the electrocardiogram signal will be described as an example with reference to FIG. 2. In the present embodiment, for ease of description, it will be described that the features of the waveform of the electrocardiogram signal shown in FIG. 2 also apply to the magnetocardiogram signal.

FIG. 2 is a diagram showing an electrocardiogram signal corresponding to a magnetocardiogram signal that is an example of a biological signal according to the embodiment. Also, the electrocardiogram signal shown in FIG. 2 is a schematic diagram for the convenience of description.

In FIG. 2, a biological signal 201, which is an electrocardiogram signal, is shown.

In the graph shown in FIG. 2, the horizontal axis represents time and the vertical axis represents a signal level (an amplitude in the example of FIG. 2).

In the graph, the biological signal 201 is shown.

On the horizontal axis of the graph, times t1 to t10 are indicated according to a direction of time progression. In the example of FIG. 2, these times t1 to t10 are not necessarily evenly spaced.

In the present embodiment, the biological signal 201 has a periodic waveform.

Also, the biological signal 201 may not necessarily have a waveform that perfectly matches each cycle. For example, although the biological signal 201 is considered to repeat the same waveform for each cycle if the state of the living body, which is a measurement target, is unchanged, the waveform of the biological signal 201 for each cycle may change when the state of the living body, which is the measurement target, changes. Also, when the state of the living body, which is the measurement target, changes, the cycle of the biological signal 201 may change.

In FIG. 2, an example of biological signals 201 for one cycle 211 and before and after the cycle 211 is shown.

Also, in the example of FIG. 2, the cycle 211 is divided into first to sixth periods 231 to 236.

The first period 231 is a period from time t1 to time t2 and is a period of a portion corresponding to a P wave of the biological signal 201.

The second period 232 is a period from time t2 to time t3 and is a period of a portion between the P wave and a QRS group (a portion corresponding to a PR segment) of the biological signal 201.

The third period 233 is a period from time t3 to time t7 and is a period of a portion corresponding to the QRS group of the biological signal 201.

In the third period 233, the biological signal 201 has a peak of the Q wave that is a minimum point at time t4, a peak of the R wave that is a maximum point at time t5, and a peak of the S wave that is a minimum point at time t6.

The fourth period 234 is a period from time t7 to time t8 and is a period of a portion between the QRS group and the T wave of the biological signal 201 (a portion corresponding to an ST segment).

The fifth period 235 is a period from time t8 to time t9 and is a period of a portion corresponding to the T wave of the biological signal 201.

Although a commonly known electrocardiogram waveform has been described as an example of a biological signal 201 having a periodic waveform in the present embodiment, an electrocardiogram interpretation method (for example, the name of each part of the waveform, a waveform separation method, or the like) is not limited. Likewise, a magnetocardiogram interpretation method (for example, the name of each part of the waveform, a waveform separation method, or the like) corresponding to this electrocardiogram is not limited.

For example, it is also possible to separate some or all of PR segments so that they are included in the P wave portion or the QRS group portion in signal processing.

For example, it is also possible to separate some or all of ST segments so that they are included in the QRS group portion or the T wave portion in signal processing.

For example, a separation method of separating the QRS group portion into finer parts in signal processing is also possible. As a specific example, a separation method for separation into a portion from time t3 to time t4, a portion from time t4 to time t6, and a portion from time t6 to time t7 or the like is also possible.

Here, a magnetocardiogram signal that is a measurement result of the magnetocardiograph is similar to an electrocardiogram signal that is a measurement result of the electrocardiograph that has been widely used conventionally and a feature of the waveform of the magnetocardiogram signal appears similar to a feature of the waveform of the electrocardiogram signal (a P wave, a QRS group, a T wave, or the like).

The cycle 211 shown in FIG. 2 corresponds to a waveform of one beat (for one cycle), which is one human heartbeat. Also, in the biological signal 201, a waveform similar to a waveform for one beat is repeated.

Although an electrocardiogram signal that is a measurement signal of an electrocardiograph is exemplified as the biological signal 201 in the present embodiment, similar features can be seen for the magnetocardiogram signal that is a measurement signal of the magnetocardiograph as described above.

Also, the present disclosure is not limited to a magnetocardiogram signal or an electrocardiogram signal as the biological signal and other signals may be used.

### <Specific examples of processing methods for each segment>

Here, a specific example of a processing method for each segment of the magnetocardiogram signal corresponding to the biological signal 201 shown in FIG. 2 is shown.

As a plurality of segments, the first period 231 including the P wave shown in FIG. 2, the third period 233 including the QRS group shown in FIG. 2, and the fifth period 235 including the T wave shown in FIG. 2 are shown as an example.

As a frequency filtering method, a frequency filtering method a1 having a filtering feature for mainly extracting a frequency component of 0.1 to 100 [Hz] and a frequency filtering method a2 having a filtering feature for mainly extracting a frequency component of 0.01 to 25 [Hz] are shown as an example.

Examples of electric current estimation calculation methods include an electric current estimation calculation method b1 using a prescribed spatial filtering method and an electric current estimation calculation method b2 using another prescribed spatial filtering method.

As a region extraction method, a region extraction method c1 for extracting a region of the atria of the heart, a region extraction method c2 for extracting regions of the atria and ventricle of the heart, and a region extraction method c3 for extracting a region of the ventricle of the heart are shown as an example.

In the first period 231, the use of the frequency filtering method a2 as the frequency filtering method is associated.

In the third period 233, the use of the frequency filtering method a1 as the frequency filtering method is associated.

In the fifth period 235, the use of the frequency filtering method a2 as the frequency filtering method is associated.

Also, in the present embodiment, in addition to the frequency filtering method associated with each segment, a process of a common notch filter may be applied in all segments. The notch filter may be a notch filter having a filtering feature for removing a frequency component of a commercial frequency (for example, 50 Hz or the like) used in a power supply.

In the first period 231, the use of the electric current estimation calculation method b2 as the electric current estimation calculation method is associated.

In the third period 233, the use of the electric current estimation calculation method b1 as the electric current estimation calculation method is associated.

In the fifth period 235, the use of the electric current estimation calculation method b2 as the electric current estimation calculation method is associated.

In the first period 231, the use of the region extraction process c1 as the region extraction process is associated.

In the third period 233, the use of the region extraction process c2 as the region extraction process is associated.

In the fifth period 235, the use of the region extraction process c3 as the region extraction process is associated.

### [Measurement unit of biological signal measurement device]

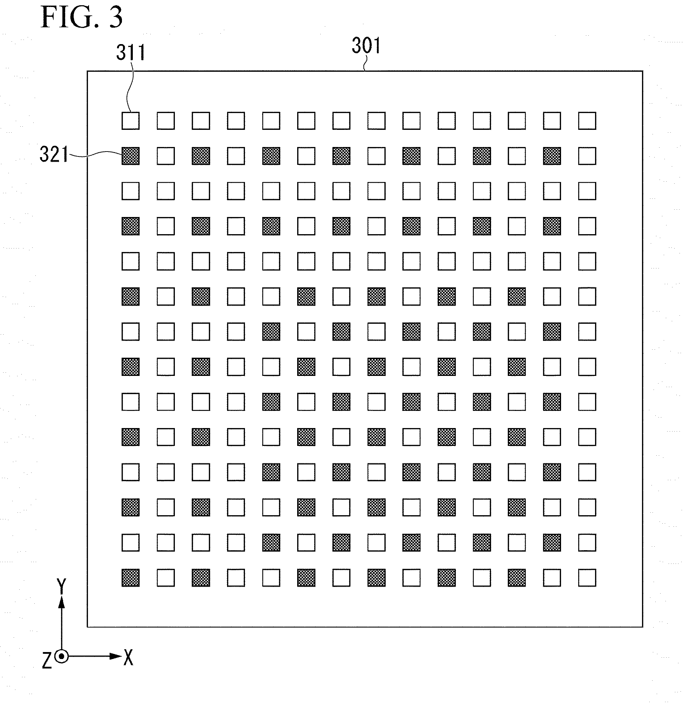

FIG. 3 is a diagram showing an example of the measurement unit 301 of the biological signal measurement device 11 according to the embodiment.

In FIG. 3, a surface of the measurement unit 301 that is facing the upper body of the human being that is an examination target is schematically shown.

In FIG. 3, an XYZ Cartesian coordinate system that is a three-dimensional Cartesian coordinate system for the convenience of description is shown. Also, in the example of FIG. 3, because it is a two-dimensional plane when focusing only on the surface of the measurement unit 301, an XY Cartesian coordinate system, which is a two-dimensional Cartesian coordinate system, may be used instead of the XYZ Cartesian coordinate system.

In the present embodiment, a total of 196 sensor accommodation units 311 in which 14 measurement units 301 are arranged at equal intervals in a prescribed direction (a direction parallel to an X-axis in the example of FIG. 3) and 14 measurement units 301 are arranged in a direction orthogonal to the direction (a direction parallel to a Y-axis in the example of FIG. 3) are provided in a matrix shape.

Also, the measurement unit 301 includes a sensor 321 accommodated in at least one of the sensor accommodation units 311.

In the present embodiment, each sensor 321 is a magnetocardiograph.

Here, the illustration is simplified so that only one sensor accommodation unit 311 is denoted by a reference numeral and only one sensor 321 is denoted by a reference numeral in the example of FIG. 3, but these are distinguished by different colors (white and black).

In the example of FIG. 3, 64 sensor accommodation units 311 among the 196 sensor accommodation units 311 include sensors 321. The biological signal detected by each sensor 321 becomes a one-channel biological signal, and the 64-channel biological signals of the 64 sensors 321 are detected in total.

For example, the sensor accommodation unit 311 is a hole provided on the surface of the measurement unit 301 and the sensor 321 is provided in the measurement unit 301 by fitting the sensor 321 into the hole.

In the example of FIG. 3, the sensor 321 can be attached to and detached from each sensor accommodation unit 311, the sensor 321 can be included in any sensor accommodation unit 311, and an arrangement pattern of a plurality of sensors 321 on the surface of the measurement unit 301 can be changed.

Also, a configuration in which the plurality of sensors 321 are included on the surface of the measurement unit 301 is not necessarily limited to the example of FIG. 3.

Although a configuration in which the sensor 321 can be provided at any location among the plurality of sensor accommodation units 311 has been shown in the example of FIG. 3 as an example, the position of the sensor 321 may be fixed as another configuration example. In this case, each sensor 321 is fixed to the measurement unit 301 and may not be detachable.

As a specific example, the surface of the measurement unit 301 may include a total of 64 sensors 321 in which 8 sensors 321 are arranged at equal intervals in a prescribed direction (for example, a direction parallel to the X-axis) and 8 sensors 321 are arranged in a direction perpendicular to the direction (for example, a direction parallel to the Y-axis) in a matrix shape.

Although a case where biological signals of 64 channels are measured simultaneously in a state in which the 64 sensors 321 are provided in the measurement unit 301 is shown in the present embodiment, the number of sensors 321 provided in the measurement unit 301 may be any value greater than or equal to 1.

Here, the biological signal has a time-series signal value in the present embodiment.

In the present embodiment, the biological signal includes measurement result information (signal values at measurement points) for a plurality of measurement points of the human upper body serving as a measurement target for the time of each point (moment). In the biological signal, the plurality of measurement points and the measurement result information (the signal values at the measurement points) are associated one-to-one for each time (measurement time).

Also, a plurality of measurement points of the human upper body of the measurement target and information representing the shape of the human upper body are associated in the present embodiment. The information representing the shape of the upper body of the human being may be included in the biological signal as an example or may be input from the user, the biological signal measurement device 11, or the like to the biological signal processing system 12 and stored in the storage unit 113 separately from the biological signal. Also, the information representing the shape of the human upper body may not necessarily be information representing a unique shape of each individual human being. For example, information representing a standard human shape may be used. In this case, the information may be input to the biological signal processing system 12 and stored in the storage unit 113 in advance.

### <Examples of measurement results>

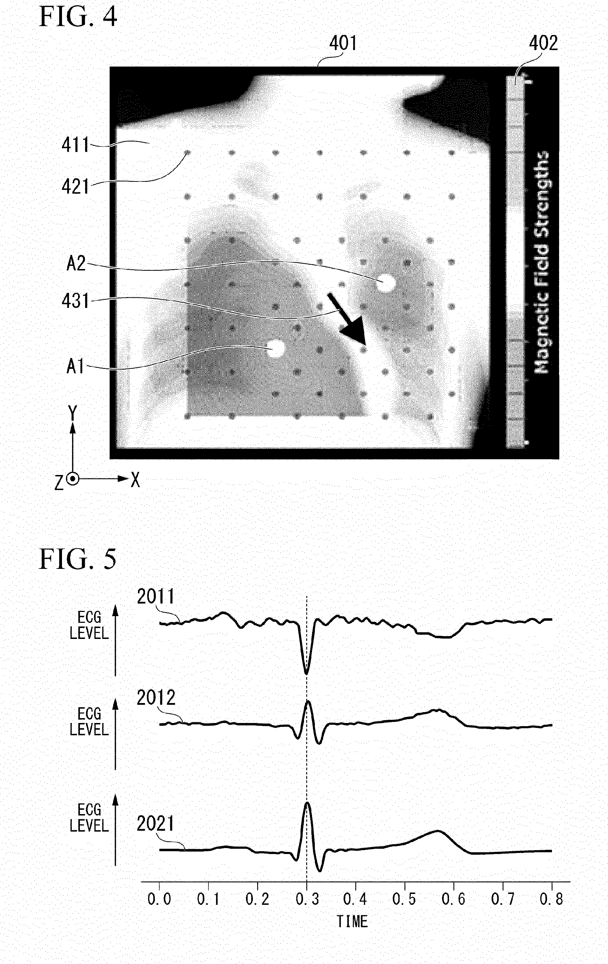

FIG. 4 is a diagram showing an example of the display of the electric current estimation calculation result according to the embodiment.

In FIG. 4, for the convenience of description, a screen 401 for displaying an example of an electric current estimation calculation result is shown.

In FIG. 4, an XYZ Cartesian coordinate system similar to that shown in FIG. 3 is shown. The XYZ Cartesian coordinate system may or may not be displayed as an example. Also, as in the example of FIG. 3, an XY Cartesian coordinate system may be used instead of an XYZ Cartesian coordinate system.

In the example of FIG. 4, the shape of the human body (an upper body 411) is shown and a position (a sensor position 421) opposite each sensor 321 and an arrow indicating an electric current estimated by the electric current estimation calculation process (an estimated current 431) is displayed.

Also, in the example of FIG. 4, the strength (magnetic field distribution) of the measured magnetic field is shown and a scale 402 is displayed.

Here, in the example of FIG. 4, the illustration is simplified so that only one sensor position 421 is denoted by a reference numeral and only one estimated current 431 is shown.

Also, the estimated current 431 is obtained as a result of analyzing the biological signal measured by the magnetocardiograph in the electric current estimation calculation method and a direction and magnitude of an electric current flowing through the human body are indicated. In the illustrated example, the direction of the current is represented by the direction of the arrow and the magnitude of the current is represented by the length of the arrow.

In FIG. 4, a position A1, which is one sensor position, and a position A2, which is another sensor position, are shown.

FIG. 5 is a diagram showing an example of the display of a magnetocardiogram signal and an electrocardiogram signal according to the embodiment. In the example of FIG. 5, for the convenience of description, a case where an electrocardiogram signal is measured simultaneously with a magnetocardiogram signal is shown.

In the example of FIG. 5, a magnetocardiogram signal 2011 measured at position A1 shown in FIG. 4 and a magnetocardiogram signal 2012 measured at position A2 shown in FIG. 4 are displayed.

Also, in the example of FIG. 5, an electrocardiogram signal 2021 measured simultaneously with these magnetocardiogram signals 2011 and 2012 is displayed.

In the graph shown in FIG. 5, the horizontal axis represents time common to all signals (the magnetocardiogram signals 2011 and 2012 and the electrocardiogram signal 2021) and the vertical axis represents a level (for example, an amplitude) for each signal.

In the example of FIG. 5, a peak appears in the vicinity of 0.3 [sec].

Although polarities (positive and negative directions) are inverted between the magnetocardiogram signal 2011 and the magnetocardiogram signal 2012, this is due to a measurement condition (for example, a measurement position).

Although magnetocardiogram signals 2011 and 2012 at two positions A1 and A2 are shown in the example of FIG. 5, magnetocardiogram signals at any one or more positions may be displayed.

### [Example of display of result of frequency filtering process]

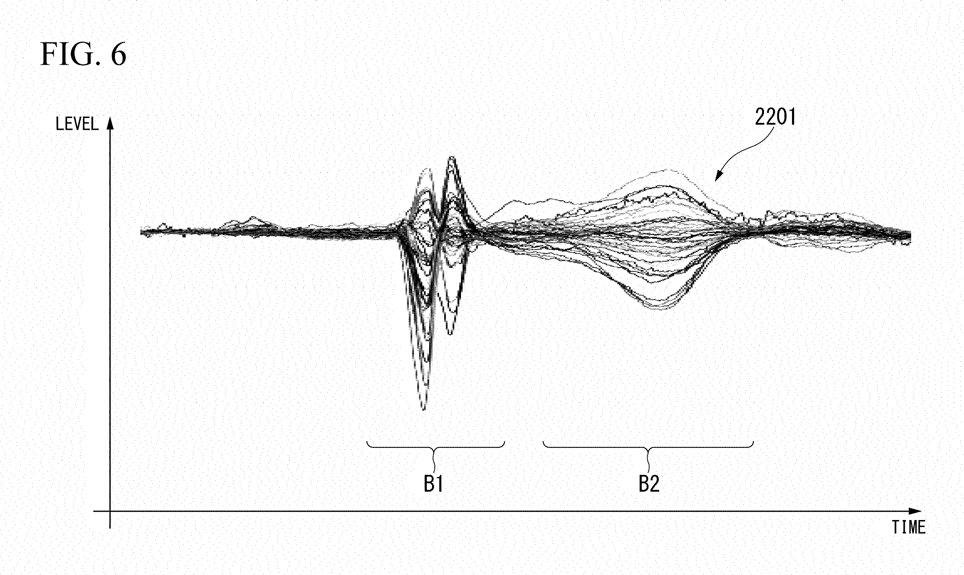

FIG. 6 is a diagram showing an example of the display of results of executing the frequency filtering process of the frequency filtering method a1 for biological signals of all segments according to the embodiment.

In the graph shown in FIG. 6, the horizontal axis represents time and the vertical axis represents a level (for example, an amplitude).

In the example of FIG. 6, a frequency-filtered signal group 2201, which is a result of executing the frequency filtering process of the frequency filtering method a1 for biological signals (magnetocardiogram signals in the present embodiment) of all segments, is displayed. The frequency-filtered signal group 2201 includes signals of 64 channels and these signal waveforms are collectively shown as the frequency-filtered signal group 2201 here.

Also, in the example of FIG. 6, a segment B1 and a segment B2, which are specific segments, are displayed. These segments B1 and B2 are segments identified by the segment identification unit 151.

Although a case where the two segments B1 and B2 are displayed is shown in the example of FIG. 6, the number of displayed segments and the displayed segments may be set arbitrarily.

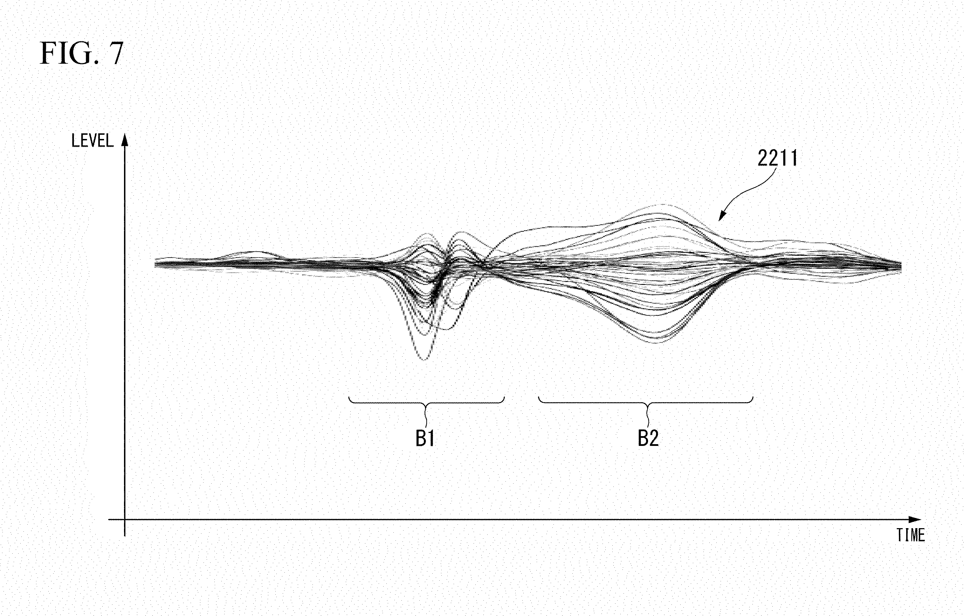

FIG. 7 is a diagram showing an example of the display of results of executing a frequency filtering process of the frequency filtering method a2 for biological signals of all segments according to the embodiment.

In the graph shown in FIG. 7, the horizontal axis represents time and the vertical axis represents a level (for example, an amplitude).

In the example of FIG. 7, a frequency-filtered signal group 2211, which is a result of executing the frequency filtering process of the frequency filtering method a2 for biological signals (magnetocardiogram signals in the present embodiment) of all segments, is displayed. The frequency-filtered signal group 2211 includes signals of 64 channels and these signal waveforms are collectively shown as the frequency-filtered signal group 2211 here.

Also, in the example of FIG. 7, a segment B1 and a segment B2, which are specific segments, are displayed. These segments B1 and B2 are segments identified by the segment identification unit 151.

Although a case where two segments B1 and B2 are displayed is shown in the example of FIG. 7, the number of displayed segments and the displayed segments may be set arbitrarily.

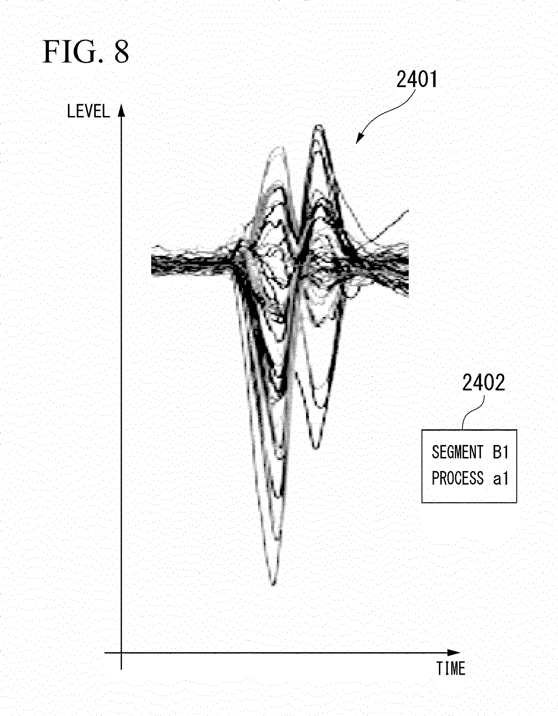

FIG. 8 is a diagram showing an example of the display of a result of executing a frequency filtering process of the frequency filtering method a1 for the biological signal of the segment B1 according to the embodiment.

In the graph shown in FIG. 8, the horizontal axis represents time and the vertical axis represents a level (for example, an amplitude).

In the example of FIG. 8, a frequency-filtered signal group 2401 is displayed only for the segment B1 shown in FIG. 6.

In the example of FIG. 8, additional information 2402 is displayed.

The additional information 2402 is information about the displayed frequency-filtered signal group 2401 and includes information indicating the segment B1 and information indicating that the frequency filtering method a1 has been used in the example of FIG. 8.

Here, content of the additional information 2402 may be arbitrary and may include various information regarding segments, processing methods, and the like as an example.

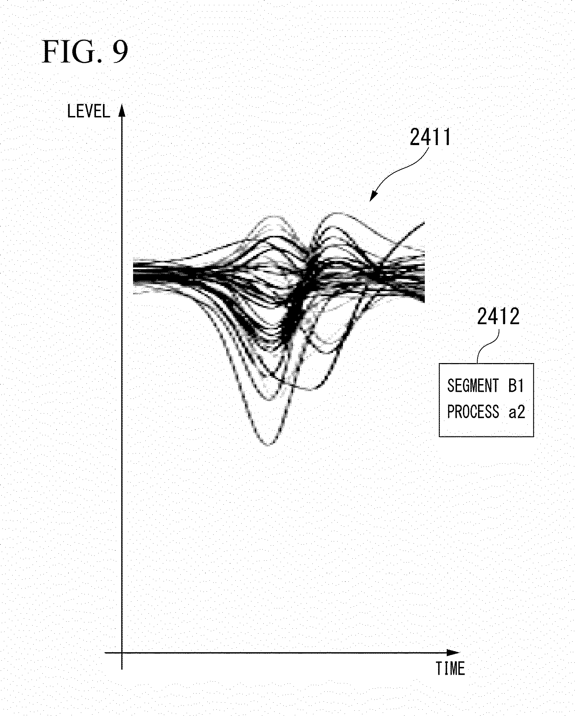

FIG. 9 is a diagram showing an example of the display of a result of executing the frequency filtering process of the frequency filtering method a2 for the biological signal of the segment B1 according to the embodiment.

In the graph shown in FIG. 9, the horizontal axis represents time and the vertical axis represents a level (for example, an amplitude).

In the example of FIG. 9, a frequency-filtered signal group 2411 is displayed only for the segment B1 shown in FIG. 7.

In the example of FIG. 9, additional information 2412 is displayed.

The additional information 2412 is information about the displayed frequency-filtered signal group 2411 and includes information indicating the segment B1 and information indicating that the frequency filtering method a2 has been used in the example of FIG. 9.

Here, content of the additional information 2412 may be arbitrary and may include, for example, various information about segments, processing methods, and the like.

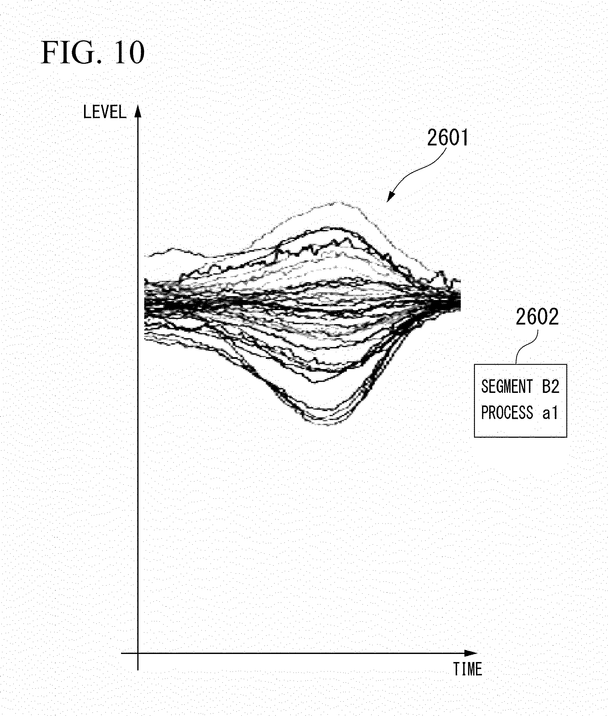

FIG. 10 is a diagram showing an example of the display of a result of executing the frequency filtering process of the frequency filtering method a1 for the biological signal of the segment B2 according to the embodiment.

In the graph shown in FIG. 10, the horizontal axis represents time and the vertical axis represents a level (for example, an amplitude).

In the example of FIG. 10, a frequency-filtered signal group 2601 is displayed only for the segment B2 shown in FIG. 6.

In the example of FIG. 10, additional information 2602 is displayed.

The additional information 2602 is information about the displayed frequency-filtered signal group 2601 and includes information indicating the segment B2 and information indicating that the frequency filtering method a1 has been used in the

### example of FIG. 10.

Here, content of the additional information 2602 may be arbitrary and may include various information regarding segments, processing methods, and the like as an example.

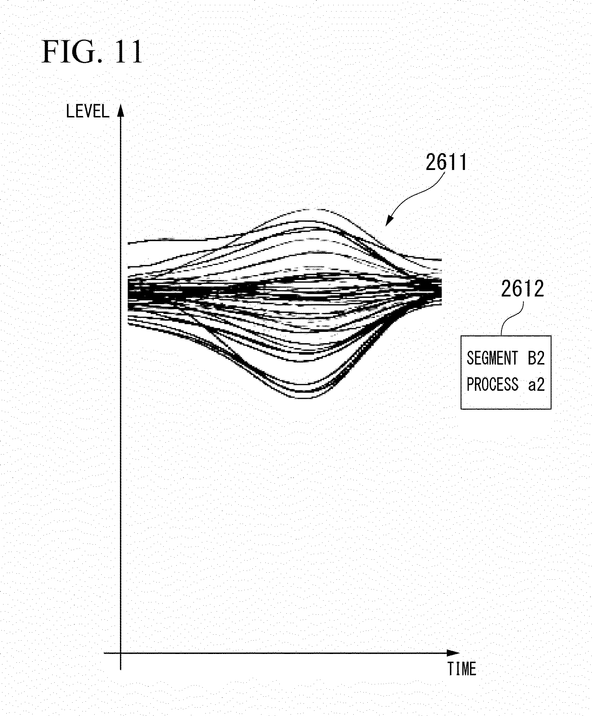

FIG. 11 is a diagram showing an example of the display of a result of executing the frequency filtering process of the frequency filtering method a2 for the biological signal of the segment B2 according to the embodiment.

In the graph shown in FIG. 11, the horizontal axis represents time and the vertical axis represents a level (for example, an amplitude).

In the example of FIG. 11, a frequency-filtered signal group 2611 is displayed only for the segment B2 shown in FIG. 7.

In the example of FIG. 11, additional information 2612 is displayed.

The additional information 2612 is information about the displayed frequency-filtered signal group 2611 and includes information indicating the segment B2 and information indicating that the frequency filtering method a2 has been used in the example of FIG. 11.

Here, content of the additional information 2612 may be arbitrary and may include various information regarding segments, processing methods, and the like as an example.

### [Example of display of result of electric current estimation calculation process]

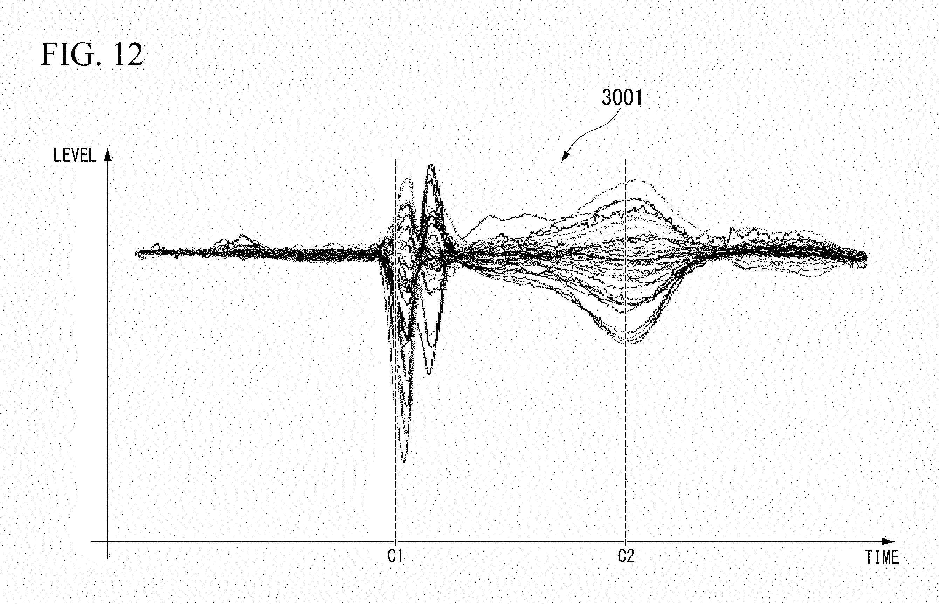

FIG. 12 is a diagram showing examples of two different times C1 and C2 in relation to the biological signal according to the embodiment.

In the graph shown in FIG. 12, the horizontal axis represents time and the vertical axis represents a level (for example, an amplitude).

In the example of FIG. 12, a frequency-filtered signal group 3001 similar to the frequency-filtered signal group 2201 shown in FIG. 6 is shown.

Also, in FIG. 12, two different times C1 and C2 are shown. Time C1 is a time included in the segment B1 of the QRS group and time C2 is a time included in the segment B2 of the T wave.

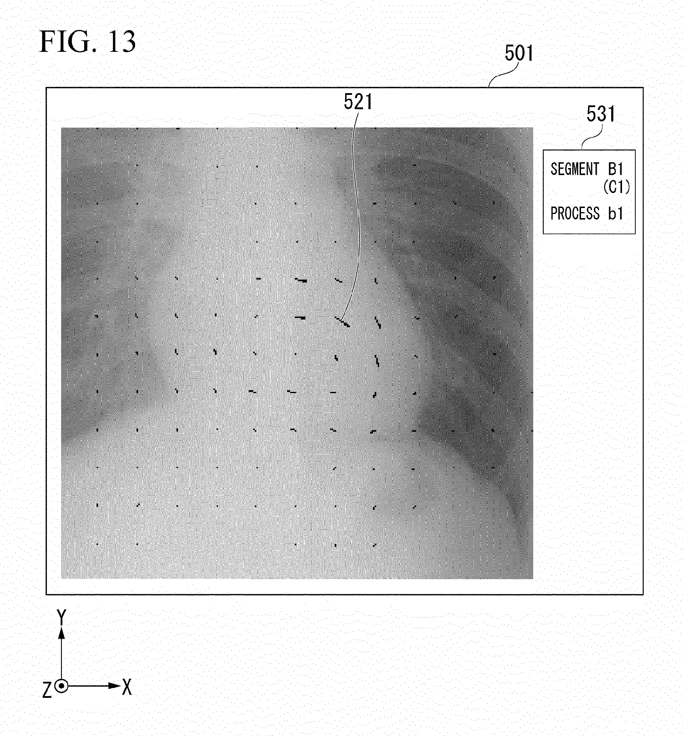

FIG. 13 is a diagram showing an example of the display of a result of executing the calculation process of the electric current estimation calculation method b1 for the biological signal of the segment B1 according to the embodiment.

In the example of FIG. 13, a screen 501 displays an example of results of executing a frequency filtering process in the frequency filtering method a1 and a calculation process in the electric current estimation calculation method b1 (calculation processing results) for the biological signal at time C1.

In FIG. 13, for the convenience of description, an XYZ Cartesian coordinate system similar to that shown in FIG. 3 is shown. The XYZ Cartesian coordinate system may or may not be displayed as an example. Also, as in the example of FIG. 3, an XY Cartesian coordinate system may be used instead of an XYZ Cartesian coordinate system.

In the example of FIG. 13, an arrow (an estimated current 521) indicating an electric current estimated in the electric current estimation calculation process is displayed with respect to an upper body region of the human body. A direction of the estimated current 521 is represented by the direction of the arrow and a magnitude of the estimated current 521 is represented by the length of the arrow.

Here, in the example of FIG. 13, the illustration is simplified and only one estimated current 521 is denoted by a reference numeral.

In the example of FIG. 13, additional information 531 is displayed.

The additional information 531 is information about the displayed calculation process result and includes information indicating the segment B1, information indicating time C1, and information indicating that the electric current estimation calculation method b1 has been used in the example of FIG. 13.

Here, content of the additional information 531 may be arbitrary and may include various information about segments, processing methods, and the like as an example.

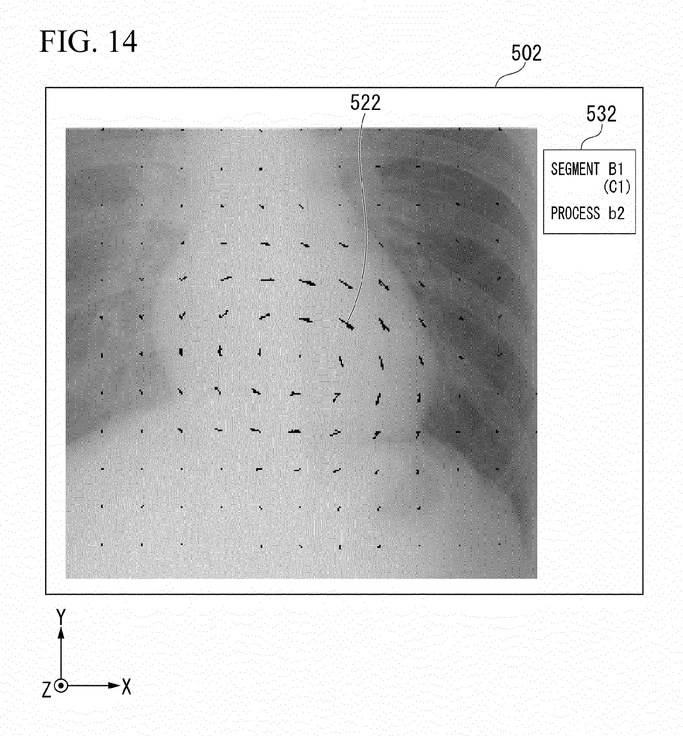

FIG. 14 is a diagram showing an example of the display of a result of executing the calculation process of the electric current estimation calculation method b2 for the biological signal of the segment B1 according to the embodiment.

In the example of FIG. 14, a screen 502 displays an example of results of executing a frequency filtering process in the frequency filtering method a1 and a calculation process in the electric current estimation calculation method b2 (calculation processing results) for the biological signal at time C1.

In FIG. 14, for the convenience of description, an XYZ Cartesian coordinate system similar to that shown in FIG. 3 is shown. The XYZ Cartesian coordinate system may or may not be displayed as an example. Also, as in the example of FIG. 3, an XY Cartesian coordinate system may be used instead of an XYZ Cartesian coordinate system.

In the example of FIG. 14, an arrow (an estimated current 522) indicating an electric current estimated in the electric current estimation calculation process is displayed with respect to an upper body region of the human body. A direction of the estimated current 522 is represented by the direction of the arrow and a magnitude of the estimated current 522 is represented by the length of the arrow.

Here, in the example of FIG. 14, the illustration is simplified and only one estimated current 522 is denoted by a reference numeral.

In the example of FIG. 14, additional information 532 is displayed.

The additional information 532 is information about the displayed calculation process result and includes information indicating the segment B1, information indicating time C1, and information indicating that the electric current estimation calculation method b2 has been used in the example of FIG. 14.

Here, content of the additional information 532 may be arbitrary and may include various information about segments, processing methods, and the like as an example.

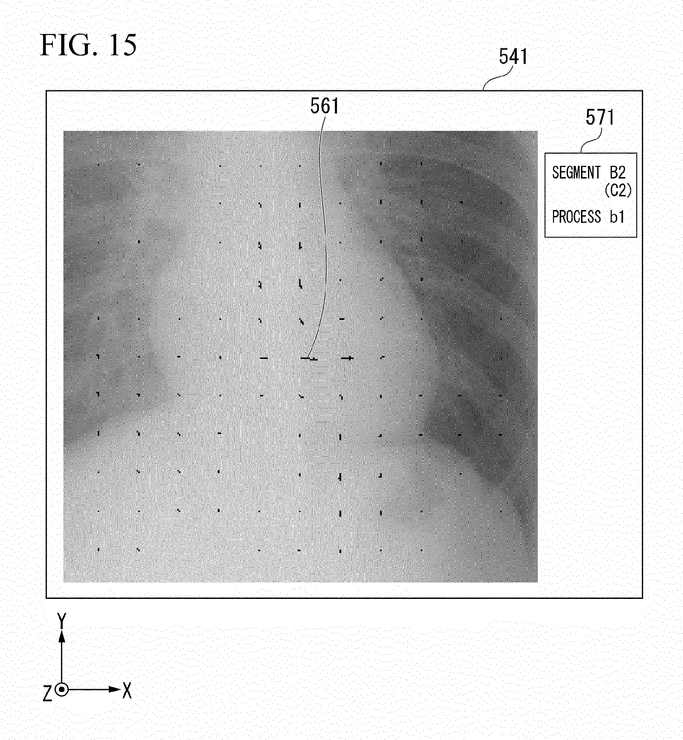

FIG. 15 is a diagram showing an example of the display of a result of executing the calculation process of the electric current estimation calculation method b1 for the biological signal of the segment B2 according to the embodiment.

In the example of FIG. 15, a screen 541 displays an example of results of executing a frequency filtering process in the frequency filtering method a1 and a calculation process in the electric current estimation calculation method b1 (calculation processing results) for the biological signal at time C2.

In FIG. 15, for the convenience of description, an XYZ Cartesian coordinate system similar to that shown in FIG. 3 is shown. The XYZ Cartesian coordinate system may or may not be displayed as an example. Also, as in the example of FIG. 3, an XY Cartesian coordinate system may be used instead of an XYZ Cartesian coordinate system.

In the example of FIG. 15, an arrow (an estimated current 561) indicating an electric current estimated in the electric current estimation calculation process is displayed with respect to an upper body region of the human body. A direction of the estimated current 561 is represented by the direction of the arrow and a magnitude of the estimated current 561 is represented by the length of the arrow.

Here, in the example of FIG. 15, the illustration is simplified and only one estimated current 561 is denoted by a reference numeral.

In the example of FIG. 15, additional information 571 is displayed.

The additional information 571 is information about the displayed calculation process result and includes information indicating the segment B2, information indicating time C2, and information indicating that the electric current estimation calculation method b1 has been used in the example of FIG. 15.

Here, content of the additional information 571 may be arbitrary and may include various information about segments, processing methods, and the like as an example.

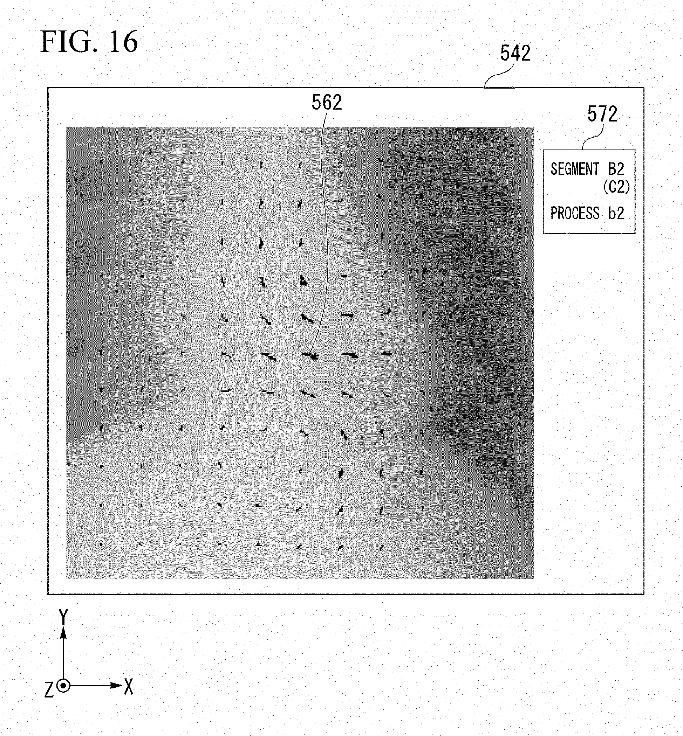

FIG. 16 is a diagram showing an example of the display of a result of executing the calculation process of the electric current estimation calculation method b2 for the biological signal of the segment B2 according to the embodiment.

In the example of FIG. 16, a screen 542 displays an example of results of executing a frequency filtering process in the frequency filtering method a1 and a calculation process in the electric current estimation calculation method b2 (calculation processing results) for the biological signal at time C2.

In FIG. 16, for the convenience of description, an XYZ Cartesian coordinate system similar to that shown in FIG. 3 is shown. The XYZ Cartesian coordinate system may or may not be displayed as an example. Also, as in the example of FIG. 3, an XY Cartesian coordinate system may be used instead of an XYZ Cartesian coordinate system.

In the example of FIG. 16, an arrow (an estimated current 562) indicating an electric current estimated in the electric current estimation calculation process is displayed with respect to an upper body region of the human body. A direction of the estimated current 562 is represented by the direction of the arrow and a magnitude of the estimated current 562 is represented by the length of the arrow.

Here, in the example of FIG. 16, the illustration is simplified and only one estimated current 562 is denoted by a reference numeral.

In the example of FIG. 16, additional information 572 is displayed.

The additional information 572 is information about the displayed calculation process result and includes information indicating the segment B2, information indicating time C2, and information indicating that the electric current estimation calculation method b2 has been used in the example of FIG. 16.

Here, content of the additional information 572 may be arbitrary and may include various information about segments, processing methods, and the like as an example.

Here, in the examples of FIGS. 13 to 16, the electric current estimation calculation method b1 is a method of detecting a large current flow and the electric current estimation calculation method b2 is a method of detecting the overall flow of an electric current. Thereby, the estimated current 522 over the entire area is visible in the example of FIG. 14 as compared with the example of FIG. 13 and the estimated current 562 over the entire area in the example of FIG. 16 is visible as compared with the example of FIG. 15. In contrast, the estimated current 521 which is strong is easily seen in the example of FIG. 13 as compared with the example of FIG. 14 and the estimated current 561 which is strong is easily seen in the example of FIG. 15 as compared with the example of FIG. 16.

### [Example of procedure of process of biological signal processing system]

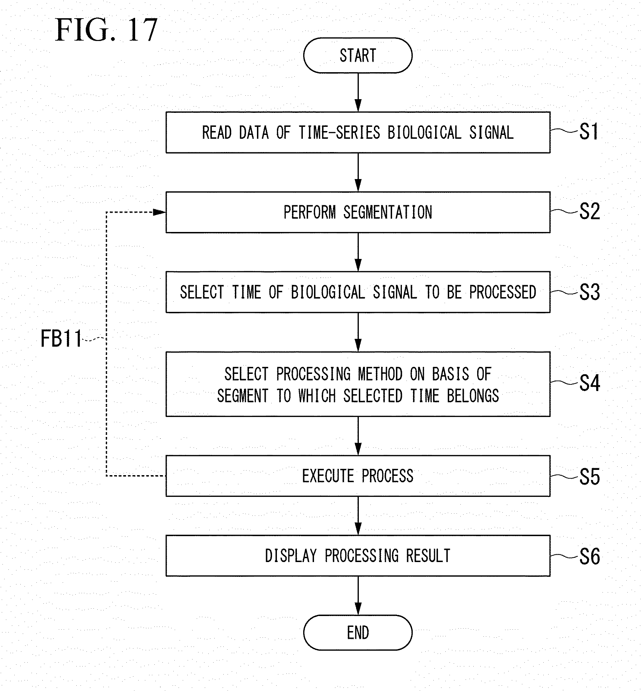

FIG. 17 is a diagram showing an example of a processing procedure performed by the biological signal processing system 12 in the biological signal measurement system 1 according to the embodiment.

In the example of FIG. 17, a case where the control unit 114 reads the entire data of the time-series biological signal (a magnetocardiogram signal in the present embodiment) in advance in the biological signal processing system 12 is shown.

### (Step S1)

The control unit 114 (for example, the segment identification unit 151) reads the entire time-series biological signal data (biological data) from the storage unit 113. In this case, the entire biological data has already been input by the input unit 111 and stored in the storage unit 113.

Also, the process proceeds to the processing of step S2.

### (Step S2)

The segment identification unit 151 divides a period of the biological signal into a plurality of segments with the segmentation unit 171 on the basis of the biological data.

Also, the process proceeds to the processing of step S3.

### (Step S3)

The segment identification unit 151 selects a time of the biological signal that is a processing target.

Also, the segment identification unit 151 identifies the segment to which the time belongs.

Also, the process proceeds to the processing of step S4.

Here, the segment identification unit 151 may select the time of the biological signal that is the processing target on the basis of a predetermined rule or select the time of the biological signal that is the processing target on the basis of an instruction by a user or the like as an example.

The rule may be, for example, a rule for selecting one or more preset times (for example, a plurality of times according to time progression) in order.

### (Step S4)

The processing control unit 191 selects a processing method on the basis of the segment identified by the segment identification unit 151 (the segment to which the selection time belongs).

Also, the process proceeds to the processing of step S5.

### (Step S5)

The process execution unit 192 executes a process for the biological signal that is the processing target in the processing method selected by the processing control unit 191.

Also, the process proceeds to the processing of step S6.

### (Step S6)

The display control unit 153 displays information about a result of the process executed by the process execution unit 192 with the display unit 141. At this time, the display control unit 153 may control a display method (a display mode) on the basis of the segment identified by the segment identification unit 151 (the segment to which the selection time belongs) or the like.

Also, the process of the present flow ends.

Here, for example, when biological data of a plurality of preset times is processed in order, the control unit 114 may move to the processing of step S3 again after the processing of step S6 is completed.

As another example, the control unit 114 may again move to the processing of step S3 when a change in the biological data that is the processing target is indicated by a user or the like after the processing of step S6 is completed.

Also, a result of the processing of step S5 may be fed back to the processing of step S2. Although such feedback FB11 is schematically shown in the example of FIG. 17, the feedback FB11 may not be performed.

### <Modified example>

A modified example of the processing flow shown in FIG. 17 is shown.

In the biological signal processing system 12, a configuration in which the control unit 114 reads the data of the biological signal in time series may be used at any time. The process of the present modified example is particularly effective in a case where a total data amount of the time-series biological signal data is large or the like as an example.

In this modified example, first, the segment identification unit 151 divides the period of the biological signal into a plurality of segments. In this modified example, the format of the biological signal data is determined and the segment identification unit 151 performs segmentation (segment definition) in advance on the basis of the format.

Also, as another configuration example, the segment identification unit 151 may perform segmentation or may use a segment identical to a segment used in a previous process as a segmentation result on the basis of biological data used in the previous process or the like.

Next, the segment identification unit 151 selects a biological signal that is the processing target (here, a signal part that is the processing target in the biological signal). This selection may be made, for example, using a waveform of the biological signal. Also, this selection may be performed, for example, using the time of the biological signal that is the processing target.

Also, the control unit 114 (for example, the segment identification unit 151) reads biological data (biological data corresponding to the time of the biological signal that is the processing target) selected from among the time-series biological signal data (biological data) from the storage unit 113. In this case, in the present modified example, the entire biological data has already been input by the input unit 111 and stored in the storage unit 113.

Thereafter, the control unit 114 performs the processing of steps S4, S5, and S6 shown in FIG. 17.

### [Other example of procedure of process of biological signal processing system]

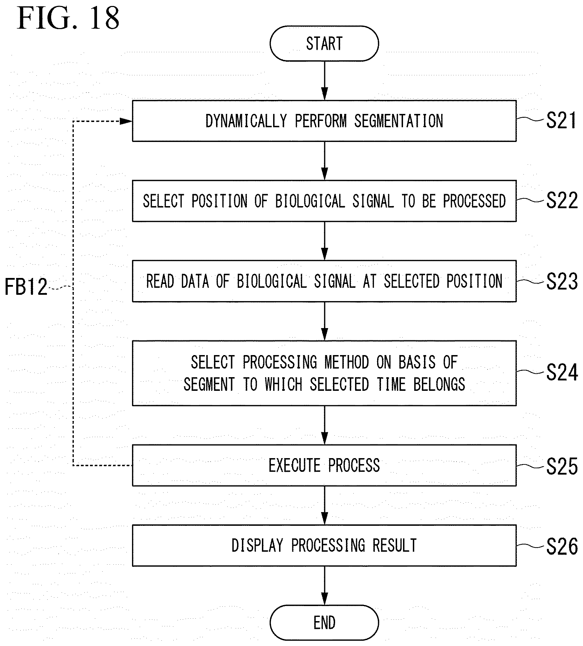

FIG. 18 is a diagram showing another example of a procedure of a process executed by the biological signal processing system 12 in the biological signal measurement system 1 according to the embodiment.

In the example of FIG. 18, a case where the biological signal processing system 12 reads a time-series biological signal (a magnetocardiogram signal in the present embodiment) from the biological signal measurement device 11 at any time in real-time is shown.

The biological signal acquisition unit 131 acquires biological signal data (biological data) as the time series progresses. For example, the storage unit 113 stores the biological signal. Information about the biological signal is information that is the processing target from now on.

### (Step S21)

The segment identification unit 151 identifies a cycle of the biological signal with the cycle identification unit 172.

In the present embodiment, the cycle identification unit 172 identifies the cycle of the biological signal on the basis of the biological signal acquired by the biological signal acquisition unit 131.

Here, when the cycle of the biological signal is known and information indicating the cycle is stored in the storage unit 113 in advance, a process of identifying the cycle may be omitted.

Also, the segment identification unit 151 divides the period of the biological signal into a plurality of segments with the segmentation unit 171 on the basis of the cycle identified by the cycle identification unit 172. The segmentation is performed dynamically.

Also, the process proceeds to the processing of step S22.

Here, when the cycle and segment of the biological signal are known and information indicating the cycle and information indicating the segment are stored in the storage unit 113 in advance, the process of step S21 may be omitted.

Also, when a segmentation method for the biological signal is known, a segmentation result is identified when the cycle is identified, and information indicating the segmentation method is stored in the storage unit 113 in advance, a segmentation process may be omitted.

### (Step S22)

The segment identification unit 151 selects a position (which may be a time point, a time, or the like) of the biological signal that is the processing target.

Also, the segment identification unit 151 identifies the segment to which the position belongs.

Also, the process proceeds to the processing of step S23.

Here, the segment identification unit 151 may select the position of the biological signal that is the processing target on the basis of a predetermined rule or may select the position of the biological signal that is the processing target on the basis of an instruction by a user or the like as an example.

The rule may be, for example, a rule for selecting one or more preset positions (for example, a plurality of positions according to time progression or the like) in order.

### (Step S23)

The biological signal processing unit 152 reads the biological signal data (biological data) at the position selected by the segment identification unit 151.

Also, the process proceeds to the processing of step S24.

### (Step S24)

The processing control unit 191 selects a processing method on the basis of the segment identified by the segment identification unit 151 (a segment to which the selection position belongs).

Also, the process proceeds to the processing of step S25.

### (Step S25)

The process execution unit 192 executes a process for the biological signal that is the processing target in a processing method selected by the processing control unit 191.

Also, the process proceeds to the processing of step S26.

### (Step S26)

The display control unit 153 displays information about a result of the process executed by the process execution unit 192 with the display unit 141. At this time, the display control unit 153 may control a display method (a display mode) on the basis of the segment identified by the segment identification unit 151 (the segment to which the selection position belongs) or the like.

Also, the process of the present flow ends.

Here, for example, when biological data of a plurality of preset positions (times) is processed in order, the control unit 114 may move to the processing of step S22 again after the processing of step S26 is completed.

As another example, after the processing of step S26 is completed, the control unit 114 may again move to the processing of step S21 when a user or the like issues an instruction for a change in the biological data that is the processing target.

Also, a result of the processing of step S25 may be fed back to the processing of step S21. Although such feedback FB12 is schematically shown in the example of FIG. 18, the feedback FB12 may not be performed.

Here, when cycle identification and segmentation are performed in the processing of step S21, they may be used fixedly or may be updated at any timing.

Specifically, the cycle identification unit 172 may identify the cycle of the biological signal at any timing and update the already identified cycle to the newly identified cycle.

As an example of a configuration, the cycle identification unit 172 identifies the cycle of the biological signal on the basis of data (biological data) different from the biological data used when the cycle of the biological signal was previously identified. Data (biological data) different from the biological data used when the cycle of the biological signal was previously identified is, for example, data that is more chronologically new than the biological data used when the cycle of the biological signal was previously specified.

As another configuration example, the cycle identification unit 172 may update the cycle to average a prescribed number of identified cycles and set an average value as a new cycle. The prescribed number of times may be any number of times. The prescribed number of times may be, for example, a sum of the latest one and the previously successively defined number of times (that is less than the prescribed number of times by 1). In the biological signal processing system 12 according to the present embodiment, the cycle may be stabilized by such averaging.

Also, the segmentation unit 171 may divide the period of the biological signal into a plurality of segments on the basis of the cycle updated by the cycle identification unit 172 and update a previous segmentation result to a new segmentation result.

As another configuration example, the segmentation unit 171 may average a prescribed number of segmentation results to set an average result as a new segment and update the segment. The prescribed number of times may be any number of times. The prescribed number of times may be, for example, a sum of the latest one and the previously successively defined number of times (that is less than the prescribed number of times by 1). In the biological signal processing system 12 according to the present embodiment, the cycle may be stabilized by such averaging.

Also, in the initial stage of the processing flow shown in FIG. 18, a provisional initial value may be set as the cycle of the biological signal, and the accuracy of the cycle may be improved by updating the initial value thereafter. In this case, the identification of the initial cycle may be omitted in the processing of step S21.

Also, in such a case, in the initial stage of the processing flow shown in FIG. 18, a provisional initial value may also be set for the segmentation result and the accuracy of segmentation may be improved by updating the initial value thereafter. In this case, the processing of step S21 may be omitted.

### [Measurement unit of biological signal measurement device according to modified example]

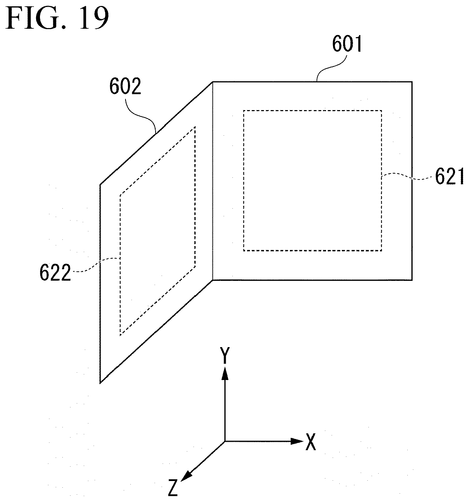

FIG. 19 is a diagram showing an example of the measurement units 601 and 602 of the biological signal measurement device according to the modified example of the embodiment.

In FIG. 19, for the convenience of description, an XYZ Cartesian coordinate system similar to that shown in FIG. 3 is shown.

In the example of FIG. 19, the two measurement units 601 and 602 having the same functions as the measurement unit 301 shown in FIG. 3 are provided.

In the example of FIG. 19, the surface of the measurement unit 601 is arranged parallel to the XY plane, and the surface of the measurement unit 602 is arranged parallel to the YZ plane. The measurement unit 601 and the measurement unit 602 are arranged so that they are orthogonal to each other and intersect each other.

In the example of FIG. 19, measurement is performed in a state in which the front of the human upper body is facing the surface of one measurement unit (for example, the measurement unit 601) and the side of the human upper body is facing the surface of the other measurement unit (for example, the measurement unit 602).

The surface of the measurement unit 601 includes a sensor area 621 that is an area where sensors of a plurality of channels are provided.

Likewise, the surface of the measurement unit 602 includes a sensor area 622 that is an area where sensors of a plurality of channels are provided.

These sensor areas 621 and 622 are similar to an area where sensors 321 of a plurality of channels are provided on the surface of the measurement unit 301 shown in FIG. 3 as an example.

By simultaneously performing measurements using the two measurement units 601 and 602 shown in FIG. 19, measurements can be performed in different directions (orthogonal to each other in the example of FIG. 19).

Also, as the number of sensors provided in each of the measurement units 601 and 602, an arrangement pattern of the sensors, or a relative arrangement of the two measurement units 601 and 602 (for example, an intersection angle), any other configuration may be used.

Also, a biological signal measurement device capable of performing measurements using three or more measurement units at the same time may be configured.

Although the biological signal measurement device 11 having a plurality of measurement units (the two measurement units 601 and 602 in the example of FIG. 19) has been described for the convenience of description in the present modified example, the plurality of measurement units may be regarded as one integrated measurement unit instead of separate measurement units.

### [Regarding above embodiments]

As described above, in the biological signal measurement system 1 according to the present embodiment, it is possible to perform an appropriate process for each feature segment of the biological signal in the biological signal processing system 12.

In the biological signal processing system 12 according to the present embodiment, for example, it is possible to perform an optimum process for each segment and increase an added value of the system by selecting a processing method corresponding to a feature of each segment (for example, a waveform or the like) so that a result of measuring the biological signal or an analysis result thereof is more accurate.

Also, in the biological signal processing system 12 according to the present embodiment, for example, the period of the biological signal can be divided into a plurality of segments in time according to a feature of the biological signal.

Also, in the biological signal processing system 12 according to the present embodiment, the processing result for each segment can be fed back for the segment identification (for example, segmentation or the like) of the segment identification unit 151.

In the present embodiment, a magnetocardiogram signal is used as a biological signal. In the waveform of the magnetocardiogram signal, there are feature segments with different amplitudes and frequencies such as a P wave, a QRS group, and a T wave. In the biological signal processing system 12 according to the present embodiment, a processing result is optimized by switching the processing method to an optimum processing method corresponding to each segment for each segment.

Here, the magnetocardiograph is expected to analyze and display various information distributed in three dimensions compared to the electrocardiograph and expected to more accurately analyze and display information distributed in space. The biological signal processing system 12 according to the present embodiment can meet such expectations, and for example, a calculation method of signal data for use in three-dimensional spatial distribution estimation can be optimized for each target waveform (each segment).

Also, in the biological signal processing system 12 according to the present embodiment, it is possible to identify the cycle of a biological signal acquired in real-time and estimate a time point of the biological signal that is a processing target on the basis of the identified cycle.

Therefore, the biological signal processing system 12 according to the present embodiment can support real-time biological signal processing.

Also, in the biological signal processing system 12 according to the present embodiment, when information such as a processing result of a certain segment is displayed, it is possible to control the display mode on the basis of the segment.

Therefore, in the biological signal processing system 12 according to the present embodiment, it is possible to display each segment in an appropriate display mode.

For example, in the biological signal processing system 12 according to the present embodiment, additional information useful to the user can be presented by displaying information for identifying the processing method applied for each segment and the like.

Also, the biological signal processing system 12 according to the present embodiment is particularly effective for processing a biological signal in which waveforms having different features for the plurality of segments (waveforms of a P wave, a QRS group, a T wave, and the like in the case of magnetocardiogram signals) are mixed as a continuous signal in which the aggregation of the plurality of segments repeats periodically over time.

### <Configuration examples>

As a configuration example, there is provided a biological signal processing system (the biological signal processing system 12 in the present embodiment) including: a segment identification unit (the segment identification unit 151 in the present embodiment) configured to identify a segment including a time point of a processing target with respect to a biological signal (for example, a magnetocardiogram signal or the like) whose period is divided into a plurality of segments in time; a processing control unit (the processing control unit 191 in the present embodiment) configured to select a processing method of processing the biological signal at the time point of the processing target (for example, one or more of a frequency filtering method, an electric current estimation calculation method, and a region extraction method) on the basis of the segment identified by the segment identification unit; and a process execution unit (the process execution unit 192 in the present embodiment) configured to execute a process for the biological signal in the processing method selected by the processing control unit.

As a configuration example, in the biological signal processing system, the processing method includes one or more of a frequency filtering method of performing a frequency filtering process, a calculation method of performing a prescribed calculation process, and a region extraction method of extracting a region that is the processing target.

As a configuration example, in the biological signal processing system, the segment identification unit includes a segmentation unit (the segmentation unit 171 in the present embodiment) configured to divide the period of the biological signal into the plurality of segments.

As a configuration example, in the biological signal processing system, the segmentation unit performs segmentation on the basis of a result of the process executed by the process execution unit.

As a configuration example, the biological signal processing system includes comprising a cycle identification unit (the cycle identification unit 172 in the present embodiment) configured to identify a cycle for the biological signal acquired in real-time. The segment identification unit estimates the time point of the processing target on the basis of the cycle identified by the cycle identification unit.

As a configuration example, the biological signal processing system includes a display unit (the display unit 141 in the present embodiment) configured to display information about a result of the process executed by the process execution unit; and a display control unit (the display control unit 153 in the present embodiment) configured to control a display mode of the display unit on the basis of the segment identified by the segment identification unit.

As a configuration example, in the biological signal processing system, the display control unit controls the display mode so that the display unit displays information about the processing method selected by the processing control unit.

As a configuration example, there is provided a biological signal measurement system (the biological signal measurement system 1 in the present embodiment) including: the biological signal processing system; and a biological signal measurement device (the biological signal measurement device 11 in the present embodiment) configured to measure the biological signal.

Also, a program for implementing the function of any component of any device described above may be recorded on a computer-readable recording medium and the program may be read and executed by a computer system. Also, the "computer system" used here may include an operating system (OS) or hardware such as peripheral devices. Also, the "computer-readable recording medium" refers to a flexible disc, a magnetooptical disc, a read-only memory (ROM), a portable medium such as a compact disc-ROM (CD-ROM), or a storage device such as a hard disk embedded in the computer system. Furthermore, the "computer-readable recording medium" is assumed to include a medium that holds a program for a constant period of time, such as a volatile memory inside a computer system serving as a server or a client when the program is transmitted via a network such as the Internet or a communication circuit such as a telephone circuit. For example, the volatile memory may be a random-access memory (RAM). For example, the recording medium may be a non-transitory recording medium.

Also, the above-described program may be transmitted from a computer system storing the program in a storage device or the like to another computer system via a transmission medium or by transmission waves in a transmission medium. Here, the "transmission medium" for transmitting the program refers to a medium having a function of transmitting information, as in a network such as the Internet or a communication circuit such as a telephone circuit.

Also, the above-described program may be a program for implementing some of the above-described functions. Further, the above-described program may be a so-called differential file capable of implementing the above-described function in combination with a program already recorded on the computer system. The differential file may be referred to as a differential program.

Also, the function of any component of any device described above may be implemented by a processor. For example, each process in the embodiment may be implemented by a processor that operates on the basis of information of a program or the like and a computer-readable recording medium that stores information of a program or the like. Here, in the processor, for example, the function of each part may be implemented by individual hardware or the function of each part may be implemented by integrated hardware. For example, the processor may include hardware and the hardware may include at least one of a circuit that processes a digital signal and a circuit that processes an analog signal. For example, the processor may be configured using one or more circuit devices or/and one or more circuit elements mounted on a circuit board. An integrated circuit (IC) or the like may be used as the circuit device and a resistor, a capacitor, or the like may be used as the circuit element.

Here, the processor may be, for example, a CPU. However, the processor is not limited to the CPU and, for example, various types of processors such as a graphics processing unit (GPU) or a digital signal processor (DSP) may be used. Also, for example, the processor may be a hardware circuit based on an application specific integrated circuit (ASIC). Also, the processor may include, for example, a plurality of CPUs, or may include a hardware circuit of a plurality of ASICs. Also, the processor may include, for example, a combination of a plurality of CPUs and a hardware circuit including a plurality of ASICs. Also, the processor may include, for example, one or more of an amplifier circuit and a filter circuit for processing an analog signal and the like.

Although embodiments of the present disclosure have been described in detail above with reference to the drawings, specific configurations are not limited to the embodiments and other designs and the like may also be included without departing from the scope of the present disclosure.

### [Reference Signs List]

1 Biological signal measurement system
11 Biological signal measurement device
12 Biological signal processing system
111 Input unit
112 Output unit
113 Storage unit
114 Control unit
131 Biological signal acquisition unit
141 Display unit
151 Segment identification unit
152 Biological signal processing unit
153 Display control unit
171 Segmentation unit
172 Cycle identification unit
191 Processing control unit
192 Process execution unit
201 Biological signal
211 Cycle
231 First period
232 Second period
233 Third period
234 Fourth period
235 Fifth period
236 Sixth period
301, 601, 602 Measurement unit
311 Sensor accommodation unit
321 Sensor
401, 501, 502, 541, 542 Screen
402 Scale
411 Upper body
421 Sensor position
431, 521, 522, 561, 562 Estimated current
621, 622 Sensor area
2011, 2012 Magnetocardiogram signal
2021 Electrocardiogram signal
2201, 2211, 2401, 2411, 2601, 2611, 3001 Frequency-filtered signal group
531, 532, 571, 572, 2402, 2412, 2602, 2612 Additional information
A1, A2 Position
B1, B2 Segment
C1, C2 Time
FB1, FB11, FB12 Feedback

## Claims

1. A biological signal processing system comprising:
a segment identification unit configured to identify a segment including a time point of a processing target with respect to a biological signal whose period is divided into a plurality of segments in time;
a processing control unit configured to select a processing method of processing the biological signal at the time point of the processing target on a basis of the segment identified by the segment identification unit; and
a process execution unit configured to execute a process for the biological signal in the processing method selected by the processing control unit.

2. The biological signal processing system according to claim 1, wherein the processing method includes one or more of a frequency filtering method of performing a frequency filtering process, a calculation method of performing a prescribed calculation process, and a region extraction method of extracting a region that is the processing target.

3. The biological signal processing system according to claim 1 or 2, wherein the segment identification unit includes a segmentation unit configured to divide the period of the biological signal into the plurality of segments.

4. The biological signal processing system according to claim 3, wherein the segmentation unit performs segmentation on a basis of a result of the process executed by the process execution unit.

5. The biological signal processing system according to any one of claims 1 to 4, comprising a cycle identification unit configured to identify a cycle for the biological signal acquired in real-time,
wherein the segment identification unit estimates the time point of the processing target on a basis of the cycle identified by the cycle identification unit.

6. The biological signal processing system according to any one of claims 1 to 5, comprising:
a display unit configured to display information about a result of the process executed by the process execution unit; and
a display control unit configured to control a display mode of the display unit on a basis of the segment identified by the segment identification unit.

7. The biological signal processing system according to claim 6, wherein the display control unit controls the display mode so that the display unit displays information about the processing method selected by the processing control unit.

8. A biological signal measurement system comprising:
the biological signal processing system according to any one of claims 1 to 7; and
a biological signal measurement device configured to measure the biological signal.
